# EUROPEAN PATENT SPECIFICATION

(11) **EP 4 591 847 B1**
(45) Date of publication and mention of the grant of the patent: **01.07.2026**
(21) Application number: 24153454.4
(22) Date of filing: 23.01.2024
(51) Int. Cl.: A61K 6/887, C08F 2/38, C08F 220/18, C08F 222/10, C08F 265/06, C09D 4/06, C08F 4/40, A61K 6/76, A61K 6/77, C08F 2/48, C08F 2/50, C08F 4/04, C08F 4/34, A61K 6/61, A61C 13/08

(54) **2-COMPONENT DENTAL MATERIALS BASED ON CROSSLINKED POLYMERIC PARTICLES BEARING ACYLTHIOUREA GROUPS**
2-KOMPONENTEN-DENTALMATERIALIEN AUF BASIS VON VERNETZTER POLYMERTEILCHEN MIT ACYLTHIOHARNSTOFFGRUPPEN
MATÉRIAUX DENTAIRES À DEUX COMPOSANTS À BASE DE PARTICULES POLYMÈRES RÉTICULÉES PORTANT DES GROUPES ACYLTHIOURÉE

(43) Date of publication of application: 30.07.2025
(73) Proprietor: Ivoclar Vivadent AG, 9494 Schaan (LI)
(72) Inventor: Catel, Yohann, 9475 Sevelen (CH); Fässler, Pascal, 7323 Wangs (CH); Schnur, Thomas, 9488 Schellenberg (LI); Lamparth, Iris, 9472 Grabs (CH)
(74) Representative: Uexküll & Stolberg

(56) References cited:
- EP-A1- 4 039 220
- US-A1- 2022 257 474
- ANONYMOUS YOHANN ET AL: "Acylthiourea oligomers as promising reducing agents for dimethacrylate-based two-component dental materials - ScienceDirect", vol. 39, no. 10, 31 October 2023 (2023-10-31), AMSTERDAM, NL, pages 886 - 893, XP093178196, ISSN: 0109-5641, Retrieved from the Internet <URL:https://www.sciencedirect.com/science/article/pii/S0109564123001628?via=ihub> [retrieved on 20240624], DOI: 10.1016/j.dental.2023.07.007
- LAMPARTH IRIS ET AL: "Polymerizable thioureas as innovative reducing agents for self-cured and dual-cured dental materials", DENTAL MATERIALS, vol. 38, no. 7, 31 July 2022 (2022-07-31), AMSTERDAM, NL, pages 1108 - 1116, XP093060957, ISSN: 0109-5641, DOI: 10.1016/j.dental.2022.04.027

## Description

The present invention relates to crosslinked polymeric particles bearing acylthiourea groups which in combination with hydroperoxides are active as initiators for the free radical polymerization, and to radically polymerizable compositions comprising the same. The crosslinked acylthiourea polymeric particles are particularly suitable for the production of dental materials, e.g., luting materials, filling composites and temporary filling materials.

In the dental field, composites are mainly used for the fabrication of direct restorations or for the cementation of indirect restorations. The polymerizable organic matrix of the composites usually consists of a mixture of monomers, initiator components, pigments and stabilizers, with mixtures of dimethacrylates often being used as monomers. Such materials can be cured by thermal, redox-initiated or light-induced radical polymerization. In addition to the organic matrix, composites contain one or more fillers, which are usually surface-modified with a polymerizable coupling agent, such as 3-methacryloyloxypropyltrimethoxysilane. Fillers improve the mechanical properties (strength, modulus of elasticity, abrasion resistance) and the processing properties (paste consistency, sculptability) of the materials and impart radiopacity.

Methacrylate-based dental materials are cured by radical polymerization using radical photoinitiators or redox initiator systems, depending on the field of application. Dual-curing systems contain a combination of photoinitiators and redox initiators.

Common photoinitiators are α-diketones such as e.g. camphorquinone (1,7,7-trimethylbicyclo[2.2.1]heptane-2,3-dione; CQ) and 9,10-phenanthrenequinone which are often used in combination with a coinitiator.

Redox initiator systems are used in dentistry to enable the self-cure of dental materials such as luting materials, composites, and temporary materials. The initiators are key components of luting materials, which are required to mediate a bond between the tooth substance and indirect restorations, such as ceramic restorations. Luting composites and self-adhesive composite cements are among the most commonly used luting materials in dentistry. These materials comprise an organic matrix, usually a mixture of dimethacrylates, an initiator system, additives, and inorganic fillers such as silanized glass fillers, spherical mixed oxides or ytterbium fluoride. They are mostly available as light-curing (LC) or dual-curing (DC) materials. Dual-curing is essential if not enough light can reach the cement through the restoration, e.g. due to its opacity, shade or thickness. The initiator system of DC luting materials contains both a photoinitiator and a redox initiator.

To ensure adequate storage stability of the redox initiators, redox initiator system-based materials are usually used as so-called 2-component (2C) systems, in which the components of the redox initiator, i.e. oxidizing agent, e.g. peroxide or hydroperoxide, and reducing agent, e.g. amines, sulfinic acids, barbiturates, thioureas are incorporated into separate components. These are mixed together just before use. For mixing, double-push syringes are increasingly being used, which have separate cylindrical chambers for holding the components. The components are pressed out of the chambers simultaneously with two connected pistons and mixed in a nozzle. To obtain mixtures that are as homogeneous as possible, it is advantageous to mix the components in approximately equal proportions by volume.

The first paste of two-component DC cements typically contains the oxidizing agent, while the second paste contains a reducing agent, and often also a metal catalyst. Upon mixing of the two pastes, a redox reaction takes place, generating radicals that initiate polymerization. The working time of the material is *inter alia* determined by the reactivity of the redox initiator system. The working time should not be too short so that the dentist can carry out the cementation before the material hardens. However, long working times are also not desirable in order to avoid unnecessary waiting time in the dental practice.

Redox-initiator systems are also used in temporary dental materials as well as in DC flowable composites for the direct filling therapy. The main advantage of DC composites is their unlimited depth of cure, as light is not mandatory for the curing of the material.

Redox initiator systems based on a mixture of dibenzoyl peroxide (DBPO) with tertiary aromatic amines, such as 4-(N,N-dimethylamino)benzoic acid ethyl ester (EDMAB), N,N-diethanol-p-toluidine (DEPT), N,N-dimethyl-sym.-xylidine (DMSX) or N,N-diethyl-3,5-di-tert-butylaniline (DABA) have been widely used to initiate radically polymerization of two-component dental materials. However, this initiator system has major drawbacks. Due to the thermal instability of DBPO, dental materials containing this peroxide must be stored in the refrigerator. In addition, the radical formation using DBPO/amine-based redox initiator systems is greatly impaired by strong acids and thus also by strongly acidic adhesive monomers which react with the amine. Moreover, oxidation of the amine can lead to discolorations of the materials. Materials comprising amines are therefore unstable.

Consequently, the dental industry intensively searched for alternative redox initiator systems that would allow the formulation of materials that can be stored at room temperature. Nowadays, hydroperoxides, such as cumene hydroperoxide (CHP) or amyl hydroperoxide, are mostly used as oxidizing agents in DC dental materials. Such hydroperoxides exhibit significantly improved thermal stability and can be stored at room temperature. On the other hand, they do not react efficiently with tertiary aromatic amines, and therefore alternative reducing agents are needed.

During the past decades, reducing agents such as (thio)barbituric acid, sulfinic acid, and ascorbic acid derivatives have been evaluated in dental materials (EP 0 480 785 A2, WO 02/092023 A1, US 2008/0014560 A1, EP 0 408 357 A2, WO 2016/007453). These reducing agents are, however, sensitive to oxygen and it is challenging to formulate storage stable materials based thereon.

For this reason, technologies based on the corresponding salts, such as sodium, calcium, potassium, ammonium salts, have been proposed (WO 2007/140440 A2). These salts are dispersed in one component of the DC cement, and an acid is added to the second component. After mixing of both components, the effective reducing agent is generated via the protonation of the corresponding salt, leading to an initiation of the polymerization.

Another redox initiator system that has been developed and used in dental materials involves urea or thiourea derivatives. US 2007/0088096 A1 discloses a redox-initiator systems based on polymerizable urea or thiourea derivatives that exhibit good acid-stability. In particular, combinations of a thiourea derivative, such as allylthiourea, with a hydroperoxide, such as potassium persulfate, are said to be stable in the presence of strongly acidic adhesive monomers.

EP 1 693 046 A1 discloses a dental composition that is said to be compatible with acidic dental primers/adhesives. The composition contains a redox initiator system that results from the combination of a (2-pyridyl)-2-thiourea derivative with a hydroperoxide compound.

WO 2007/016508 A1 discloses dental compositions that contain a thiourea/hydroperoxide combination as an initiator system. Those compositions do not contain acid-functional compounds.

According to EP 1 754 465 A1, the reactivity of the thiourea/hydroperoxide system can be increased by adding a soluble copper compound.

EP 2 233 544 A1 describes two-component dental compositions that contain a redox initiator system based on the combination of a thiourea derivative, a hydroperoxide and a vanadium compound as a polymerization accelerator.

Although DC dental materials based on the thiourea and hydroperoxide system are storage stable, they still have drawbacks. In particular, thiourea derivatives have a bitter taste (D. Mela, Chem. Senses 14 (1989) 131-135; Lange et al., J. Amer. Chem. Soc. 51 (1929) 1911-1914; Qin et al., Talanta 199 (2019) 131-139) that can still be perceived after curing, which many patients find unpleasant.

EP 4 039 220 A1 discloses radically polymerizable dental materials wherein the redox system comprises a hydroperoxide and an acylthiourea derivative comprising a radically polymerizable group. The materials can additionally contain a transition metal compound. On curing, the acylthiourea derivatives are incorporated into the polymer network. If the acylthiourea derivatives do not polymerize completely, residual monomers can be leached out of the material and cause a bitter taste. Dental Materials Volume 39, Issue 10, October 2023, Pages 886-893 relates to acylthiourea oligomers (ATUO1-ATUO3), prepared as follow, as promising reducing agents for dimethacrylate-based two-component dental materials

It is an object of the invention to provide radically polymerizable materials which do not have the disadvantages of the state of the art and which are particularly suitable as dental materials. The materials must not taste bitter and should have good biocompatibility. In addition, the materials should exhibit excellent mechanical properties (flexural strength and flexural modulus) as well as an appropriate working time.

The object is achieved by a redox initiator system that comprises a combination of a hydroperoxide, crosslinked polymeric particles that bear acylthiourea groups and optionally a transition metal compound.

The crosslinked polymeric particles are obtainable via the copolymerization, in the presence of a chain transfer agent, of at least one difunctional monomer, preferably at least one monofunctional monomer and at least one thiourea derivative according to the following Formula (I): 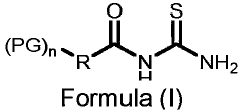 in which the variables have the following meanings:
- R: is an (n+1)-valent, aromatic, aliphatic, linear or branched C₁-C₅₀ hydrocarbon radical, which can be interrupted by one or more ether, thioether, ester, amide or urethane groups,
- PG: a radically polymerizable (meth)acrylate, (meth)acrylamide or vinyl group,
- n: is 1 or 2.

The variables of Formula (I) preferably have the following meanings:
- R: an (n+1)-valent, aromatic, aliphatic, linear or branched C₁-C₃₀ hydrocarbon radical, which can be interrupted by one or more ether, ester or urethane groups,
- PG: a radically polymerizable (meth)acrylate or vinyl group and
- n: is 1 or 2.

The variables particularly preferably have the following meanings:
- R: an (n+1)-valent, aliphatic, linear or branched C₁-C₂₀ hydrocarbon radical, which can be interrupted by one or more ether, ester or urethane groups,
- PG: a radically polymerizable (meth)acrylate group and
- n: is 1 or 2.

The preferred, more preferred and most preferred definitions given for the individual variables can be selected in each case independently of each other. Compounds in which all the variables have the preferred, more preferred or most preferred definitions are naturally particularly suitable according to the invention.

All formulae shown herein extend only to those compounds which are compatible with the theory of chemical valence. The indication that a radical is interrupted e.g. by one or more ether groups is to be understood to mean that these groups are inserted in each case into the carbon chain of the radical. These groups are thus bordered on both sides by C atoms and cannot be terminal. C₁ radicals cannot be interrupted. Corresponding to the usual nomenclature, by aromatic hydrocarbon radicals is also meant those radicals which contain aromatic and non-aromatic groups. A preferred aromatic radical is, for example, -Ph-CH₂-.

According to a preferred embodiment of the present invention, the crosslinked polymeric particles are obtained by the copolymerization of a monomer mixture comprising:
(1) 10 to 80 mol%, preferably 15 to 60 mol% and more preferably 20 to 50 mol% of the at least one thiourea derivative according to Formula (I),
(2) 10 to 80 mol%, preferably 15 to 70 mol% and more preferably 20 to 60 mol% of the at least one difunctional monomer and
(3) 10 to 80 mol%, preferably 15 to 70 mol% and more preferably 20 to 20 mol% of the at least one monofunctional monomer,
based on the total molar amount of monomers.

The copolymerization is carried out in the presence of at least one chain transfer agent. The one ore more transfer agents are preferably added to the above mixture of monomers (1) to (3) in a total amount of at least 10 mol%, more preferably 10 to 30 mol%, even more preferably 10 to 25 mol% and most preferably 10 to 20 mol%, based on the total molar amount of monomers (1) to (3).

The crosslinked polymeric particles bearing acylthiourea groups are synthesized via free-radical polymerization, preferably by suspension polymerization, emulsion polymerization and more preferably by solution polymerisation. The polymerization is preferably carried out using thermal free-radical polymerization in the presence of a suitable initiator. Preferred thermal initiators according to the invention are azo radical initiators such as azobis(isobutyronitrile) (AIBN), 2,2'-azobis[2-methyl-N-(2-hydroxyethyl)propionamide], 4,4'-azobis(4-cyanovaleric acid), 1,1'-azobis(cyclohexanecar-bonitrile), 2,2'-azobis[2-(2-imidazolin-2-yl)propane] dihydrochloride, 2,2'-azobis(2-methylpropionamidine)dihydrochloride), 2,2'-azobis (2,4-dimethylvaleronitrile), 2,2'-azobis(2-methylbutyronitrile) or dimethyl 2,2'-azobis(2-methylpropionate), diperoxides, in particular dibenzoyl peroxide, dilauroyl peroxide, di-tert-butyl peroxide, and peroxodi-sulfates, in particular potassium peroxodisulfate K₂S₂O₈, sodium peroxodisulfate Na₂S₂O₈. Alternatively, the crosslinked polymeric particles can be synthesized by redox polymerization or by photopolymerization.

The acylthiourea (meth)acrylates of formula (I) can be easily prepared according to synthetic pathways that are described in EP 4 039 220 A1.

Polymerizable thiourea derivatives of Formula (I) preferred according to the invention are:

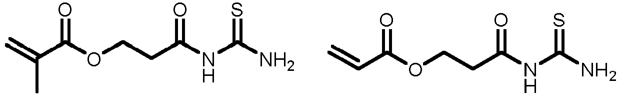

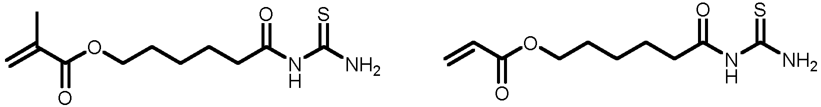

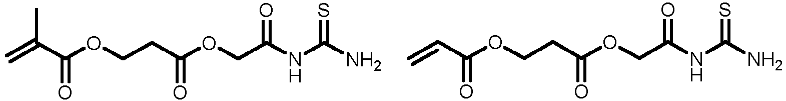

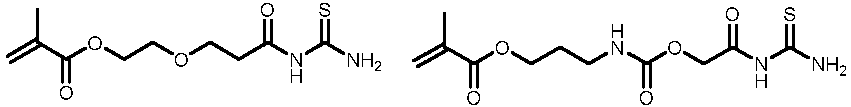

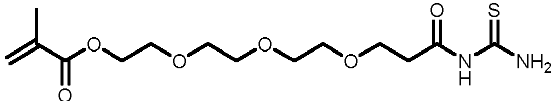

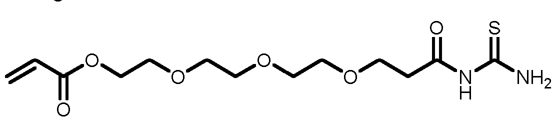

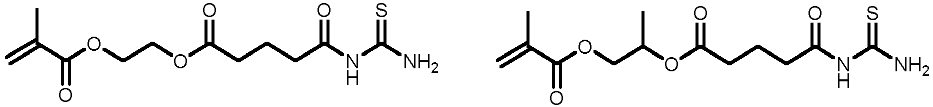

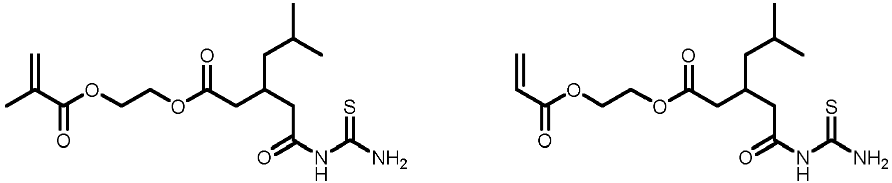

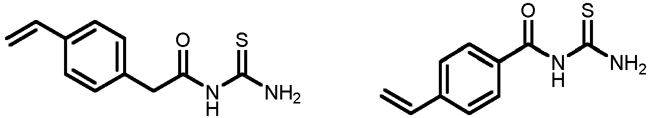

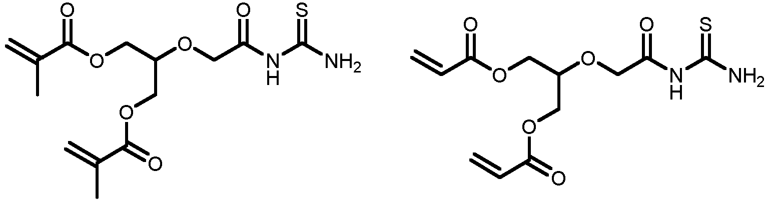

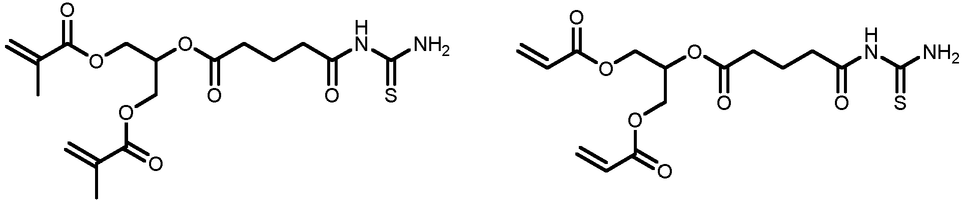

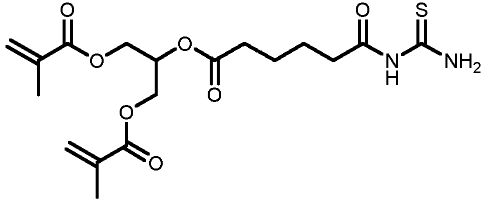

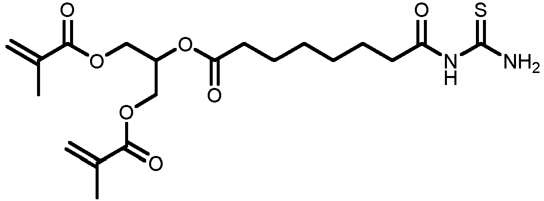

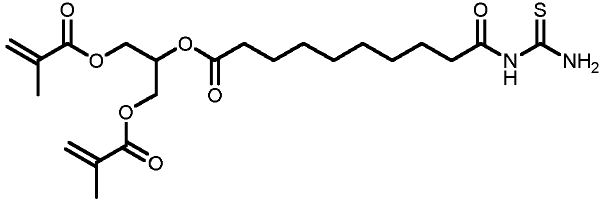

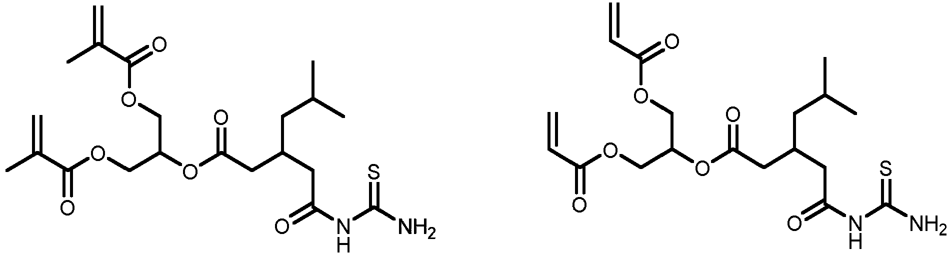

**Monofunctional monomers** refer to molecules bearing a single polymerizable group and no thiourea moieties. Monofunctional monomers such as (meth)acrylates, (meth)acrylamides, N,N-dialkyl(meth)acrylamides, styrene and derivatives thereof, vinyl acetate and N-vinylpyrrolidone are preferred for the synthesis of the crosslinked polymeric particles.

Monofunctional (meth)acrylates are more preferably used for the synthesis of the crosslinked polymeric particles. Aromatic (meth) acrylates such as 2-phenoxyethyl (meth)acrylate, benzyl (meth)acrylate, p-Cumylphenoxyethylene glycol (meth)acrylate and 2-(2-Biphenyloxy)-ethyl (meth)acrylate can be used. Cycloaliphatic monofunctional (meth)acrylates such as isobornyl (meth)acrylate, furfuryl (meth)acrylate, tetrahydrofurfuryl (meth)acrylate, dicyclopentadienyl (meth)acrylate and 4-tert-butylcyclohexyl (meth)acrylate are preferred. Preferred monofunctional monomers are also (meth)acrylates bearing a functional group such as 2-hydroxyethyl (meth)acrylate, 2-hydroxypropyl (meth)acrylate as well as glycol (meth)acrylates such as ethylene glycol methyl ether (meth)acrylate and di(ethylene glycol) methyl ether (meth)acrylate. (Meth)acrylates bearing a urethane group are also preferred. They can be easily synthesized, as an example, via the reaction of a hydroxyalkyl (meth)acrylate with an isocyanate or via the reaction of an alcohol with the 2-isocyanatoethyl methacrylate. Mostly preferred comonomers are aliphatic linear or branched alkyl (meth)acrylates such as methyl (meth)acrylate, ethyl (meth)acrylate, propyl (meth)acrylate, butyl (meth)acrylate, 2-ethylhexyl (meth)acrylate. Methacrylates are preferred over acrylates.

**Difunctional monomers** refer to molecules bearing two polymerizable groups and no thiourea moieties. Difunctional monomers such as di(meth)acrylates, di(meth)acrylamides and di-N,N-dialkyl(meth)acrylamides are preferred for the synthesis of the cross-linked polymeric particles.

Difunctional (meth)acrylates are more preferably used for the synthesis of the cross-linked polymeric particles. Preferred difunctional (meth)acrylates are bisphenol A dimethacrylate, bis-GMA (an addition product of methacrylic acid and bisphenol A diglycidyl ether), ethoxylated or propoxylated bisphenol A di(meth)acrylate, such as the bisphenol A dimethacrylate SR-348c (Sartomer) with 3 ethoxy groups or 2,2-bis[4-(2-methacryloyloxypropoxy)phenyl]propane, UDMA (an addition product of 2-hydroxyethyl methacrylate and 2,2,4-trimethylhexamethylene-1,6-diisocyanate), 2-{[(2-(meth-acryloyloxy)ethoxy)carbonyl]amino} ethyl methacrylate, tetramethylxylylene diurethane di(meth)acrylates, such as tetramethylxylylene diurethane ethylene glycol di(meth)acrylate and tetramethylxylylene diurethane-2-methylethylene glycoldi(meth)acrylate and in particular monomer V380, di-, tri- or tetra-ethylene glycol di(meth)acrylate, glycerol di(meth)acrylate, 1,4-butanediol di(meth)acrylate, 1,6-hexanediol di(meth)acrylate, 1,10-decanediol di(meth)acrylate, bis(methacryloyloxymethyl)tricyclo-[5.2.1.02,6]decane (DCP), polyethylene glycol or polypropylene glycol di(meth)acrylates, such as polyethylene glycol 200-dimethacrylate or polyethylene glycol 400-dimethacrylate (PEG-200- or PEG-400-DMA) or 1,12-dodecanediol di(meth)acrylate.

Preferred tetramethylxylylene diurethane di(meth)acrylates are monomers according to the following formula:

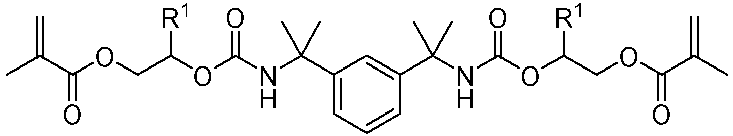

In this formula, the R¹ groups are each independently H or CH₃, and the R¹ residues may have the same meaning or different meanings. Preferably, a mixture is used which contains molecules in which both residues are H, molecules in which both residues are CH₃, and molecules in which one residue is H and the other residue is CH₃, the ratio of H to CH₃ preferably being 7:3. Such a mixture is obtainable, for example, by reaction of 1,3-bis(1-isocyanato-1-methylethyl)benzene with 2-hydroxypropyl methacrylate and 2-hydroxyethyl methacrylate. This mixture is referred to herein as monomer V380.

**Chain transfer agents (CTA)** are used in order to control the degree of cross-linking of the cross-linked polymeric particles. Chain transfer agents are also called chain regulators. Preferred chain transfer agents are mercaptans, in particular mercaptans of the formula R²-(SH)_{z}, in which R² is a linear or branched aliphatic C₁-C₁₅ hydrocarbon radical, which can be interrupted by one or more S and/or O atoms and/or -COO-groups and can be unsubstituted or substituted by one or more -Br and/or -Cl atoms and/or -OH or -COOH groups, or an aromatic C₆-C₁₂ hydrocarbon radical, and z is 1 or 2, preferably 1. R² is preferably a branched and particularly preferably a linear C₂-C₁₅ alkyl radical, which can be substituted by functional groups, in particular -Br, -Cl, -OH and/or -COOH, and which is preferably not substituted. Particularly preferred mercaptans are lauryl mercaptan (1-dodecanethiol, DDT), 2-mercaptoethanol, 3-mercaptopropanol, 3-mercapto-2-butanol, 2-mercapto-3-butanol, 3-mercapto-2-methyl-butan-1-ol, 3-mercapto-3-methylhexan-1-ol, 3-mercaptohexanol, 2-mercaptoacetic acid, 3-mercaptopropionic acid, 4-methylbenzenethiol, isooctyl 3-mercaptopropionate, tert-nonylmercaptan, 4,4'-thiobisbenzenethiol and 1,8-dimercapto-3,6-di-oxaoctane.

Further preferred chain transfer agents are disulfides (R³-S-S-R³), in particular dithiourethane disulfides, such as tetramethylthiuram disulfide and isopropylxanthic disulfide. Disulfides are also called photoiniferters because they act as photoinitiator (photoini-) during the radical photopolymerization and at the same time also take part in transfer reactions (-fer-) and termination reactions (-ter).

Also preferred as chain transfer agents are addition-fragmentation chain transfer (AFCT) agents, such as vinyl ethers, allyl sulfides, allyl sulfones, allyl halides and allyl phosphonates.

The most preferred chain transfer agents according to the present invention are alkyl mercaptans according to the formula R²-SH, wherein R² is a linear C₂-C₁₅ alkyl radical, mercaptoethanol and mercaptoacetic acid.

The crosslinked acylthiourea polymeric particles according to the invention, in combination with a hydroperoxide compound, are active as initiators for the radical polymerization. The crosslinked acylthiourea polymeric particles and the hydroperoxides together form a redox initiator system. The present invention also relates to a redox initiator system for the radical polymerization comprising crosslinked acylthiourea polymeric particles according to the invention and at least one hydroperoxide, and to radically polymerizable compositions which in addition to the crosslinked acylthiourea polymeric particles and the at least one hydroperoxide comprise at least one radically polymerizable monomer. According to a further preferred embodiment, the initiator system and radically polymerizable composition additionally comprise a transition metal compound.

It is a particular advantage of the crosslinked acylthiourea polymeric particles described in this invention that they are not released from the cured materials after polymerization of the polymerizable composition. They are physically and chemically bound into the polymer network. This is a significant advantage in terms of bitterness properties and possible toxic side effects. The acylthiourea polymer particles of the invention are therefore particularly suitable for dental and other medical applications.

The initiator systems and polymerizable compositions of the present invention contain at least one **hydroperoxide compound.** Preferred hydroperoxides are compounds of the formula R⁴-(OOH)_{y}, in which R⁴ is an aliphatic or aromatic hydrocarbon radical and y is 1 or 2. Preferred radicals R⁴ are alkyl and aryl groups. The alkyl groups can be straight-chain, branched or cyclic. Cyclic alkyl radicals can be substituted by aliphatic alkyl groups. Alkyl groups with 4 to 10 carbon atoms are preferred. Aryl groups can be unsubstituted or substituted by alkyl groups. Preferred aromatic hydrocarbon radicals are benzene radicals which are substituted with 1 or 2 alkyl groups. The aromatic hydrocarbon radicals preferably contain 6 to 12 carbon atoms. Particularly preferred hydroperoxides are t-amyl hydroperoxide, 1,1,3,3-tetramethylbutyl hydroperoxide, t-butyl hydroperoxide, t-hexyl hydroperoxide, 2,5-dimethyl-2,5-di(hydroperoxy)hexane, diisopropylbenzene monohydroperoxide, paramenthane hydroperoxide, p-isopropylcumene hydroperoxide and mixtures thereof. Cumene hydroperoxide (CHP) is quite particularly preferred.

Further preferred are the low-odor CHP derivatives of Formula (II) disclosed in European patent application EP 3 692 976 A1: 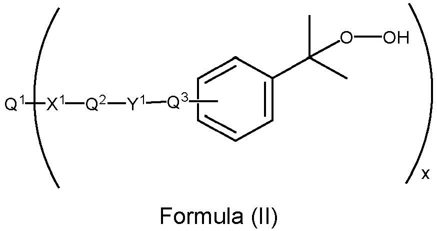
in which the variables have the following meanings:
   - Q¹: a x-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR⁵, -Cl and -Br, wherein R⁵ is an aliphatic, linear or branched C₁-C₁₀ hydrocarbon radical,
   - X¹, Y¹: independently of each other are in each case absent, -O-, -COO-; -CONR⁶-, or -O-CO-NR⁷-, wherein R⁶ and R⁷ independently of each other represent H or a C₁-C₅ alkyl radical, preferably H, methyl and/or ethyl, particularly preferably H, and wherein X¹ and Y¹ are preferably not absent at the same time and wherein X¹ and/or Y¹ is absent if Q² is absent,
   - Q²: is absent, an aliphatic, linear or branched C₁-C₁₄ alkylene radical, which can be interrupted by S and/or O atoms and which can be unsubstituted or substituted by -OH, -OR⁸, -Cl and/or -Br, wherein R⁸ is an aliphatic, linear or branched C₁-C₁₀ hydrocarbon radical,
   - Q³: a C₁-C₃ alkylene group or is absent, preferably -CH₂- or is absent,
   - x: 1, 2, 3 or 4,
and wherein the substitution on the aromatic ring takes place in position 2, 3 or 4, relative to the cumene hydroperoxide group.

The variables of Formula (II) preferably have the following meanings:
- Q¹: a mono- or divalent, aliphatic, linear or branched C₁-C₁₀ hydrocarbon radical, which can be interrupted by one or more O atoms, preferably one O atom, and which can be substituted by one or more, preferably one, substituents which are selected from -OH and -OR⁵ or is preferably unsubstituted, wherein R⁵ is an aliphatic, linear or branched C₁-C₆ hydrocarbon radical,
- X¹, Y¹: independently of each other are in each case absent, -O-, -COO- or -O-CO-NR⁷-, wherein R⁷ represents H or a C₁-C₅ alkyl radical, preferably H, methyl and/or ethyl and quite particularly preferably H, and wherein X¹ and Y¹ are preferably not absent at the same time,
- Q²: is absent, a linear or branched C₁-C₁₀ alkylene radical, which can be interrupted by one or more O atoms, preferably one O atom, and which can be substituted by one or more, preferably one, substituents which are selected from -OH and - OR⁸ or is preferably unsubstituted, wherein R⁸ is an aliphatic, linear or branched C₁-C₆ hydrocarbon radical,
- x: 1 or 2,
and wherein the substitution on the aromatic ring takes place in position 3, preferably in position 4.

More preferably the variables of Formula (II) have the following meanings:
- Q¹: a mono- or divalent, aliphatic, linear or branched C₁-C₅ hydrocarbon radical, which can be interrupted by one O atom and which can be substituted by one OH group,
- X¹: -COO-,
- Y¹: is absent,
- Q²: is absent or a linear C₁-C₃ alkylene radical,
- x: 1 or 2,
and wherein the substitution on the aromatic ring takes place in position 4.

Most preferably the variables of Formula (II) have the following meanings:
- Q¹: a mono- or divalent, aliphatic, branched, preferably linear C₁-C₄ hydrocarbon radical,
- X¹: -COO-,
- Y¹: is absent,
- Q²: is absent with or a methylene radical,
- x: 1 or 2, and wherein the substitution on the aromatic ring takes place in position 4.

Hydroperoxide derivatives of Formula (II) particularly preferred according to the invention are:

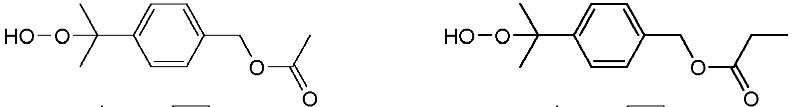

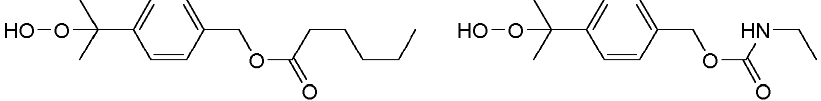

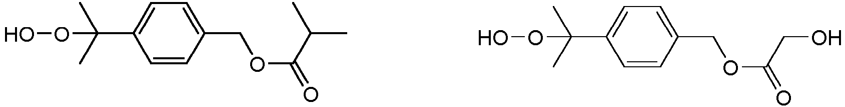

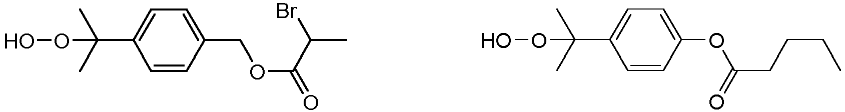

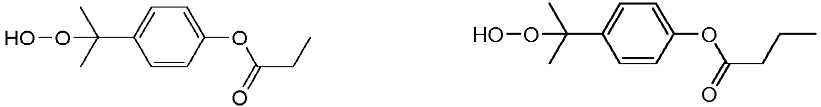

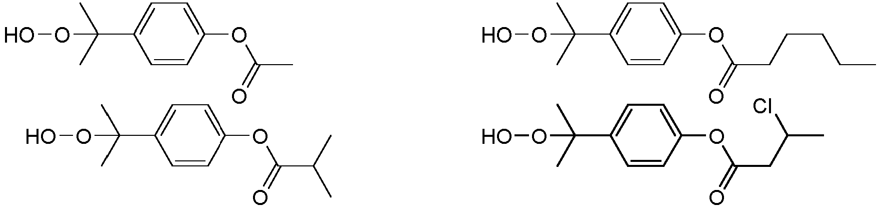

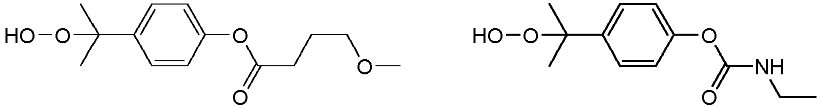

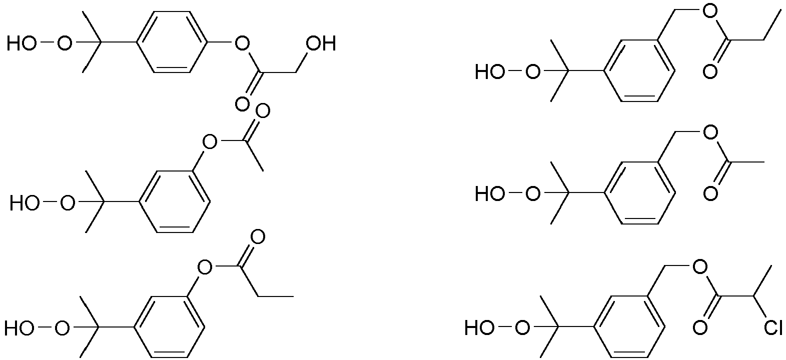

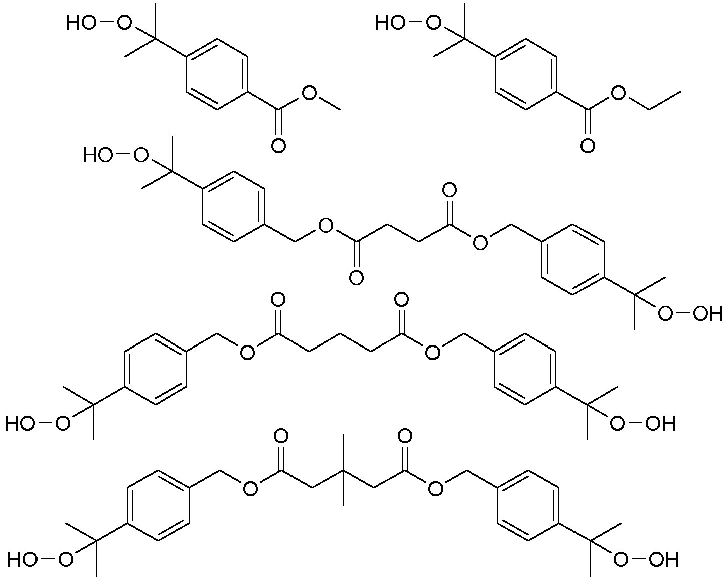

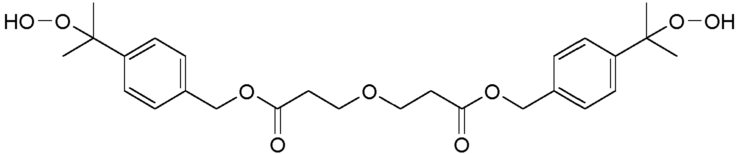

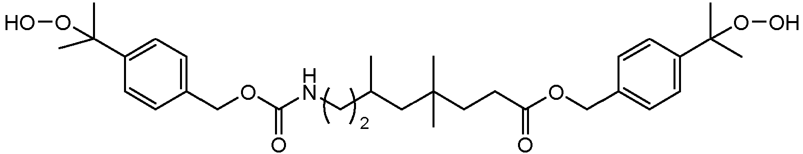

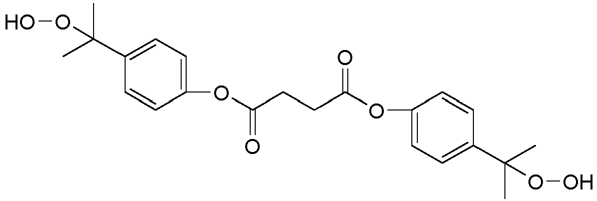

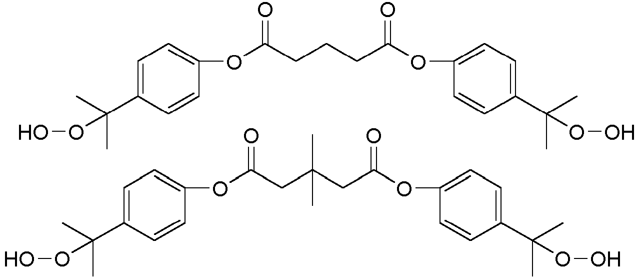

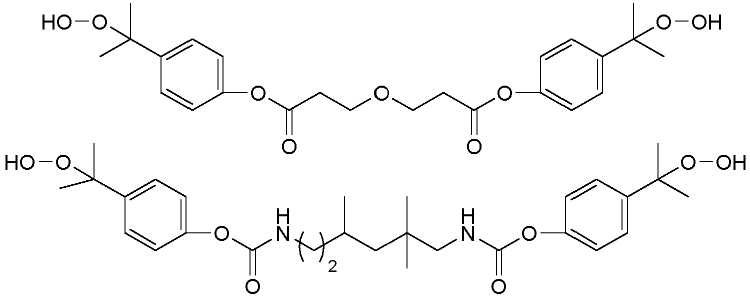

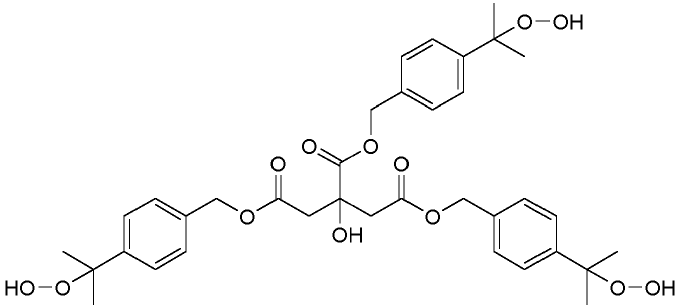

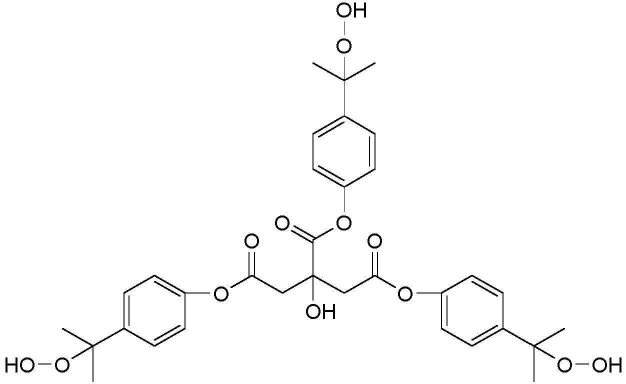

The hydroperoxide derivatives of Formula (II) have a low odor and high storage stability at room temperature. They are therefore particularly suitable for the production of low-odor dental materials. The initiator systems and polymerizable compositions of the present invention can contain one or more hydroperoxides.

The initiator systems and polymerizable compositions of the present invention preferably contain at least one **transition metal compound.** It was found that the addition of a transition metal compound yields materials which have significantly improved mechanical properties after hardening.

Preferred transition metal compounds according to the invention are compounds which are derived from transition metals which have at least two stable oxidation states. Compounds of the elements copper, cobalt, nickel, vanadium and manganese are particularly preferred. These metals have the following stable oxidation states: Cu(I)/Cu(II), Fe(II)/Fe(III), Co(II)/Co(III), Ni(II)/Ni(III), V(III)/V(IV), V(IV)/V(V), Mn(II)/Mn(III). Materials which contain at least one copper compound are particularly preferred. In all cases, those compounds in which the respective transition metal is present in its most stable oxidation state are preferred. Compounds of Cu²⁺ are thus most preferred.

The transition metals are preferably used in the form of their salts. Preferred salts are the nitrates, acetates, 2-ethylhexanoates and halides, wherein chlorides are particularly preferred.

The transition metals can furthermore advantageously be used in complexed form, wherein complexes with chelate-forming ligands are particularly preferred. Preferred simple ligands for complexing the transition metals are 2-ethylhexanoate and THF. Preferred chelate-forming ligands are 2-(2-aminoethylamino)ethanol, aliphatic amines, particularly preferably 1,1,4,7,10,10-hexamethyltriethylenetetramine (HMTETA), N,N,N',N",N"-pentamethyldiethylenetriamine (PMDETA), tris[2-(dimethylamino)ethyl]-amine (Me₆TREN), N,N,N',N'-tetramethylethylenediamine (TMEDA), 1,4,8,11-tetraaza-1,4,8,11-tetramethyl-cyclotetradecane (Me4CYCLAM), diethylenetriamine (DETA), triethylenetetramine (TETA) and 1,4,8,11-tetraazacyclotetradecane (CYCLAM); pyridine-containing ligands, particularly preferably N,N,N',N'-tetrakis(2-pyridylmethyl)ethylenediamine (TPEN), N, N-bis(2-pyridylmethyl)amine (BPMA), N,N-bis(2-pyridylmethyl)octylamine (BPMOA), 2,2'-bipyridine and 8-hydroxyquinoline. Most particularly preferred ligands are acetylacetone, dimethylglyoxime and 1,10-phenanthroline. Metal complexes that are soluble in the respective monomer mixture are particularly preferred.

In the case of electrically neutral ligands, the charge of the transition metal ions must be balanced by suitable counterions. For this, the above-named ions which are used to form salts are preferred wherein acetates and chlorides are particularly preferred. Chlorides and complexes are characterized by a relatively good solubility in monomers, which are used to prepare dental materials.

Instead of the transition metal complexes, non-complex salts of the transition metals in combination with complex-forming organic compounds can be used to prepare the dental materials, preferably in combination with the above-named chelate-forming compounds. The organic ligands form the catalytically active complexes when mixed with the transition metal salts. The use of such combinations of transition metal salts and organic ligands is preferred.

Preferred copper salts are CuCl, CuBr, CuCl₂, CuBr₂, CuI₂, Cu(II) carboxylates (e.g. of acetic acid or 2-ethylhexanoic acid). Preferred copper complexes are complexes with the ligands acetylacetone, phenanthroline (e.g. 1,10-phenanthroline (phen)), the aliphatic amines, such as e.g. 1,1,4,7,10,10-hexamethyltriethylenetetramine (HMTETA), N,N,N',N",N"-pentamethyldiethylenetriamine (PMDETA), tris[2-(dimethylamino)ethyl]-amine (Me₆TREN).

Preferred iron salts are FeCl₃, FeBr₂ and FeCl₂. Preferred iron complexes are complexes with the ligands acetylacetone, triphenylphosphine, 4,4'-di(5-nonyl)-2,2'-bipyridine (dNbpy) or 1,3-diisopropyl-4,5-dimethylimidazol-2-ylidene (Prilm). The complexes Fe(acac)₂ and FeCl₂(PPh₃)₂ are quite particularly preferred.

Preferred nickel salts are NiBr₂ and NiCl₂, preferred nickel complexes are nickel acetylacetonate and NiBr₂(PPh₃)₂.

According to the invention, copper compounds, copper complexes and in particular mixtures of copper salts and complexing organic ligands are particularly preferred.

Initiators systems and polymerizable compositions which contain crosslinked acylthiourea polymeric particles according to the invention, at least one hydroperoxide derivative and at least one transition metal compound, wherein these components are in each case selected from the above-defined preferred and particularly preferred substances, are quite particularly preferred.

The crosslinked acylthiourea polymeric particles according to the invention are used in an amount of from 0.2 to 10 wt.%, preferably 0.3 to 8.0 wt.% and more preferably 0.5 to 5.0 wt.%.

The hydroperoxide compound is preferably used in an amount of from 0.1 to 5.0 wt.%, more preferably 0.2 to 4.0 wt.% and most preferably 0.3 to 3.0 wt.%.

The transition metal compound is preferably used in an amount of from 0.0001 to 0.5 wt.%, more preferably 0.0005 to 0.1 wt.% and most preferably 0.001 to 0.020 wt.%.

Unless otherwise stated, all weight percentages herein relate to the total mass of the polymerizable composition.

The initiator system and the polymerizable compositions according to the invention can advantageously further comprise an **initiator for the radical photopolymerization.** Such compositions are dual-curing, i.e. they can be cured both chemically and by light. Photoinitiators can be added to the catalyst paste, the base paste or both.

Preferred photoinitiators are alpha-diketones derivatives, preferably camphorquinone (CQ), 9,10-phenanthrenequinone, 1-phenylpropane-1,2-dione, 2,2-dimethoxy-2-phenylacetophenone, diacetyl or 4,4'-dichlorobenzil or derivatives thereof. Camphorquinone (CQ), 2,2-dimethoxy-2-phenylacetophenone and mixtures thereof are most particularly preferred. Alpha-diketones are preferably used in combination with tertiary amines as coinitiator. Preferred coinitiators are 4-(dimethylamino)benzoic acid ester (EDMAB), N,N-dimethylaminoethyl methacrylate, N,N-dimethyl-sym.-xylidine and triethanolamine. Bimolecular photoinitiators, i.e. photoinitiators that require a second component to reach their optimal activity, are referred to as Norrish type 2 photoinitiators.

Norrish type 1 photoinitiators can also be used. The materials according to the invention preferably contain one or more Norrish type 1 photoinitiator, more preferably such a photoinitiator which is active in a wavelength range of from 400 to 500 nm.

Preferred Norrish type 1 photoinitiators are monoacyl- or bisacylphosphine oxides, diacyldialkylgermanium and tetraacylgermanium compounds as well as tetraacylstannanes. Particularly preferred monomolecular photoinitiators are 2,4,6-trimethylbenzoyl diphenylphosphine oxide, bis(2,4,6-trimethylbenzoyl) phenylphosphine oxide, ethyl (2,4,6-trimethylbenzoyl) phenylphosphinate (TPO-L), dibenzoyldiethylgermane, bis(4-methoxybenzoyl)diethylgermanium (MBDEGe, trade name Ivocerin), tetrabenzoylgermane, tetrakis(o-methylbenzoyl)germane, tetrakis(mesitoyl)stannane and mixtures thereof.

Moreover, mixtures of the different photoinitiators can also be used, such as e.g. bis(4-methoxybenzoyl)diethylgermane or tetrakis(o-methylbenzoyl)germane in combination with camphorquinone and 4-dimethylaminobenzoic acid ethyl ester.

The radically polymerizable compositions of the present invention comprise at least one multifunctional (meth)acrylate and may optionally comprise one or more monofunctional (meth)acrylates. The multifunctional (meth)acrylates preferably comprise 2 to 4 radically polymerizable groups and the monofunctional (meth)acrylates one radically polymerizable group. Neither multifunctional nor monofunctional monomers comprise thiourea moieties.

Preferred **multifunctional (meth)acrylates** are bisphenol A dimethacrylate, bis-GMA (an addition product of methacrylic acid and bisphenol A diglycidyl ether), ethoxylated or propoxylated bisphenol A dimethacrylate, such as the bisphenol A dimethacrylate SR-348c (Sartomer) with 3 ethoxy groups or 2,2-bis[4-(2-methacryloyloxypropoxy)-phenyl]propane, UDMA (an addition product of 2-hydroxyethyl methacrylate and 2,2,4-trimethylhexamethylene-1,6-diisocyanate), 2-{[(2-(methacryloyloxy)ethoxy)carbonyl]-amino} ethyl methacrylate, tetramethylxylylene diurethane di(meth)acrylates, such as tetramethylxylylene diurethane ethylene glycol di(meth)acrylate and tetramethylxylylene diurethane-2-methylethylene glycoldi(meth)acrylate and in particular monomer V380, di-, tri- or tetraethylene glycol dimethacrylate, trimethylol propanetrimethacrylate, pentaerythritol tetramethacrylate and glycerol di- and trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol or polypropylene glycol dimethacrylates, such as polyethylene glycol 200-dimethacrylate or polyethylene glycol 400-dimethacrylate (PEG-200- or PEG-400-DMA) or 1,12-dodecanediol dimethacrylate. UDMA, V-380, 2-{[(2-(methacryloyloxy)ethoxy)carbonyl]amino} ethyl methacrylate, bisphenol A dimethacrylate SR-348c (Sartomer) and PEG-400-DMA (NK ester 9G) are particularly preferred.

Preferred **monofunctional monomers** are benzyl and furfuryl methacrylate, 2-phenoxyethyl methacrylate, 2-(o-biphenyloxy)ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]-ethyl methacrylate, 1-phenoxypropan-2-yl methacrylate and 2-(p-cumylphenoxy)-ethyl methacrylate, tricyclodecane methacrylate, tricyclodecane methyl methacrylate and/or 2-(p-cumylphenoxy)ethyl methacrylate, and in particular cumylphenoxyethylene glycol methacrylate (CMP-1E).

According to one embodiment, the compositions according to the invention contain one or more **acid-group-containing radically polymerizable monomers** (adhesive monomers) in addition to the above-named monomers. These give the materials self-adhesive and/or self-etching properties. Acid-group-containing monomers are therefore particularly suitable for the preparation of self-adhesive dental materials, such as e.g. luting cements.

Preferred acid-group-containing monomers are polymerizable carboxylic acids, phosphonic acids and phosphoric acid esters as well as their anhydrides. Preferred carboxylic acids and carboxylic acid anhydrides are 4-(meth)acryloyloxyethyl trimellitic acid anhydride, 10-methacryloyloxydecylmalonic acid, N-(2-hydroxy-3-methacryloyl-oxypropyl)-N-phenylglycine, 4-vinylbenzoic acid. Preferred phosphoric acid esters are 2-methacryloyloxyethylphenyl hydrogen phosphate, 10-methacryloyloxydecyl dihydrogen phosphate (MDP) and dipentaerythritol pentamethacryloyloxyphosphate. Preferred phosphonic acids are 4-vinylbenzyl-phosphonic acid, 2-[4-(dihydroxyphos-phoryl)-2-oxa-butyl]-acrylic acid and their amides, esters, such as e.g. 2-[4-(dihydroxy-phosphoryl)-2-oxa-butyl]-acrylic acid-2,4,6-trimethylphenyl ester.

It is known that the addition of acidic monomers to two-component composites based on the hydroperoxide/thiourea redox system results in a significant reduction of the working time. Therefore, the amount of redox components is often reduced to lower the reactivity, but this leads to a deterioration of the mechanical properties. For this reason, it is challenging to formulate two-component self-adhesive cements that have both excellent mechanical properties and an adequate working time. It was surprisingly found, that the crosslinked polymer particles bearing acylthiourea groups according to the present invention allow the formulation of 2C materials having a favourable working time without impairing the mechanical properties.

The polymerizable compositions according to the invention can moreover advantageously contain one or more **organic or inorganic fillers.** Particulate fillers are preferred. Filler-containing compositions are particularly suitable as dental fixing cements or filling composites.

Preferred inorganic fillers are oxides, such as SiO₂, ZrO₂ and TiO₂ or mixed oxides of SiO₂, ZrO₂, ZnO and/or TiO₂, nanoparticulate or microfine fillers, such as pyrogenic silica or precipitated silica, glass powders, such as quartz, glass ceramic, borosilicate, or radiopaque glass powders, preferably barium or strontium aluminum silicate glasses or fluoroaluminosilicate glasses, and radiopaque fillers, such as ytterbium trifluoride, tantalum(V) oxide, barium sulfate or mixed oxides of SiO₂ with ytterbium(III) oxide or tantalum(V) oxide. The dental materials according to the invention can furthermore contain fibrous fillers, nanofibers, whiskers or mixtures thereof.

Preferably, the oxides have a particle size of from 0.010 to 15 µm, the nanoparticulate or microfine fillers preferably have a particle size of from 10 to 300 nm, the glass powders preferably have a particle size of from 0.01 to 15 µm, more preferably of from 0.2 to 1.5 µm, and the radiopaque fillers preferably have a particle size of from 0.2 to 5 µm.

Particularly preferred fillers are mixed oxides of SiO₂ and ZrO₂, with a particle size of from 10 to 300 nm, glass powders with a particle size of from 0.2 to 1.5 µm, in particular fluoroaluminosilicate glass powders and radiopaque glass powders, preferably of barium or strontium aluminium silicate glasses, and radiopaque fillers with a particle size of from 0.2 to 5 µm, in particular ytterbium trifluoride and/or mixed oxides of SiO₂ with ytterbium(III) oxide.

Preferred radiopaque glass fillers have the following composition (wt.%): SiO₂: 20-80; B₂O₃: 2-15, BaO or SrO: 10-40; Al₂O₃: 2-20; CaO and/or MgO: 0-20; Na₂O, K₂O, Cs₂O: 0-10 each; WO₃: 0-20; ZnO: 0-20; La₂O₃: 0-10; ZrO₂: 0-15; P₂O₅: 0-30; Ta₂O₅, Nb₂O₅ or Yb₂O₃: 0-5; and CaF₂ and/or SrF₂ 0-10. Particularly preferred are radiopaque glass fillers having the composition (wt.%): SiO₂: 40-75; B₂O₃: 2-15; BaO or SrO: 15-35; Al₂O₃: 2-15; CaO and/or MgO: 0-10; and Na₂O: 0-10.

Preferred fluoroaluminosilicate (FAS) glass fillers have the following composition (wt.%): SiO₂: 20-35; Al₂O₃: 15-35; BaO or SrO: 10-25; CaO: 0-20; ZnO: 0-15; P₂O₅: 5-20; Na₂O, K₂O, Cs₂O: 0-10 each; and CaF₂: 0.5-20. Particularly preferred are FAS fillers with the composition (wt.%): SiO₂: 20-30; Al₂O₃: 20-30; BaO or SrO: 10-25; CaO: 5-20; P₂O₅: 5-20; Na₂O: 0-10; and CaF₂: 5-20.

The FAS fillers and radiopaque glass fillers preferably have an average particle size of 0.2 to 20 µm and particularly preferably of 0.4 to 5 µm.

According to a preferred embodiment of the invention, the fluoroaluminosilicate glass filler and/or radiopaque glass filler has been pre-treated with acid. By the acid treatment of the filler, the storage stability of two-component materials containing acidic monomers can be significantly improved. Preferred acid treated fillers and their preparation are described in European Patent Application No. EP 4 205 724 A1.

The acid treatment of the particulate FAS or glass fillers is carried out by washing the filler with acid, preferably by the following process:
(i) The particulate FAS or glass filler is dispersed in an aqueous solution of an organic or preferably inorganic acid. The acid is preferably used in a concentration of 0.1 to 5 mol/l, particularly preferably 0.5 to 3 mol/l.
(ii) The dispersion from step (i) is then stirred, preferably for 0.5 to 24 h, more preferably for 1 to 5 h.
(iii) After the stirring in an acid solution, the filler is separated and washed with deionized water. For this purpose, it is preferably dispersed in water and stirred for 1 to 60 minutes, particularly preferably for 2 to 20 minutes.
(iv) After washing, the filler is separated and dried, preferably in a vacuum drying oven in a fine vacuum at 20 to 80 °C, particularly preferably at 40 to 60 °C. The filler is preferably dried to constant weight.
(v) After drying, the filler is preferably subjected to an optional thermal treatment. For this purpose, it is preferably heated for 2 to 12 h, particularly preferably for 4 to 6 h, to a temperature that is well below the glass transition temperature of the filler, preferably 200 to 500 °C and particularly preferably 300 to 400 °C.

The acid used to treat the fillers is preferably completely removed from the fillers after the acid treatment. The fillers are not coated with an acid.

The acid treatment can be repeated once or several times. Steps (i) and (iii) are preferably carried out at a temperature in the range from 5 to 50°C, particularly preferably at room temperature (23°C). The temperature is measured in the solution or dispersion in each case.

Acids that form soluble salts with Ca, Al, Sr and Ba ions are preferred. Particularly preferred are formic acid, acetic acid and especially hydrochloric acid, and nitric acid. Alternatively, acids that form sparingly soluble salts with Ca, Al, Sr or Ba ions, such as phosphoric acid, may be used but these are less preferred. Poorly soluble salts are defined as salts with a solubility of less than 0.1 g/l (in water at room temperature). According to the invention, acidic organic monomers and acidic organic polymers as well as peracids and hydrofluoric acid are not suitable as acids.

The washing step (iii) is preferably repeated 1 to 5 times, particularly preferably 3 times, so that the acid is completely removed from the filler. For this purpose, the filler is separated from the water following step (iii) and again dispersed and stirred in deionized water. The washing is repeated until the pH of the water in the last washing step is ≥ 5. After washing, the filler can be subjected to further acid treatment and washing. The sequence of acid treatment and washing can be repeated once or several times.

The acid treatment of the fillers significantly improves the storage stability of the compositions according to the invention. In particular, the content of radical polymerizable monomers comprising an acid group decreases only slowly, so that the compositions show high adhesion to the tooth structure and particularly to dentin even after prolonged storage. The acid treatment thus significantly improves the properties of dental composites with self-adhesive properties in a simple manner.

According to another preferred embodiment of the invention, the fluoroaluminosilicate glass fillers and/or radiopaque glass fillers are preferably used in combination with at least one sulfate and/or phosphate which is water-soluble at 20°C. Water-soluble sulfates or phosphates were found to improve the storage stability of compositions comprising a fluoroaluminosilicate glass filler and/or radiopaque glass filler in combination with acidic monomers. The fluoroaluminosilicate glass filler and/or radiopaque glass filler may be used in acid-treated or non-acid-treated form. Preferred fillers comprising a fluoroaluminosilicate glass and/or radiopaque glass in combination with at least one water-soluble sulfate and/or phosphate and their preparation are described in European Patent Application No. EP 4 205 723 A1.

Preferred sulfates are inorganic salts of sulfuric acid and preferred phosphates are inorganic salts of orthophosphoric acid (H₃PO₄). Water-soluble sulfates or phosphates are sulfates or phosphates which preferably have a water solubility of at least 100 g/l, preferably of 110 to 1,000 g/l and particularly preferably of 150 to 800 g/l. Preferred water-soluble sulfates are potassium sulfate (K₂SO₄; water solubility 111 g/l), sodium sulfate (Na₂SO₄; water solubility 170 g/l) and particularly preferably ammonium sulfate ((NH₄)₂SO₄; water solubility 754 g/l). Preferred water-soluble phosphates are potassium phosphate (K₃PO₄; water solubility 508 g/l), sodium phosphate (Na₃PO₄; water solubility 285 g/l) and especially preferably ammonium phosphate ((NH₄)₃PO₄; water solubility 580 g/l). All solubility data refer to solubility in water at 20°C.

The one or more water-soluble sulfates and/or water-soluble phosphates are preferably added in a total amount of at least 0.3 wt.% and, more preferably, at least 0.4 wt.%. According to the invention, compositions are preferred which contain up to 0.3 to 9.0 wt.%, more preferably 0.4 to 6.0 wt.%, and most preferably from 0.7 to 4.0 wt.% of at least one water-soluble sulfate and/or phosphate. Unless otherwise stated, all percentages herein are based on the total mass of the composition. The sulfates or phosphates may be present in dissolved or preferably in solid form. According to the invention, sulfates are preferred.

According to still another preferred embodiment of the invention, the fluoroaluminosilicate glass filler (FAS filler) and/or radiopaque glass filler is coated with a silica (hetero)polycondensate. Preferred coated fillers and their preparation are described in European Patent Application No. 22202860.7.

The term silica (hetero)polycondensate stands for silica polycondensate or silica hetero polycondensate. Surprisingly, it was found that coating the filler with a silica (hetero)polycondensate results in a significant increase in the storage stability of the compositions without deteriorating the other properties. The coating of the fillers prevents significant amounts of metal ions, in particular Al, Ca, Ba or Sr ions, from diffusing from dental fillers, such as radiopaque glass fillers and FAS fillers, into the resin mixture and reacting there with acidic monomers. It also prevents acidic monomers from binding to the filler surface by forming insoluble salts. This avoids a decrease in the concentration of acid monomers in the resin matrix and a consequent deterioration in adhesion, and improves the storage stability of the materials.

The filler is preferably coated by a sol-gel process. For this purpose, a sol is prepared by hydrolytic condensation of one or more condensable compounds of silicon and optionally other elements from the group boron, aluminum, titanium or phosphorus and/or precondensates derived from these compounds, brought into contact with the filler and the filler is subsequently dried.

Preferred hydrolytically condensable compounds of silicon are silanes of the general Formula (V):

SiX²ₐR⁹₄₋ₐ Formula (III)

in which the variables have the following meanings:
- R⁹ =: C₁-C₂₀-alkyl or C₂-C₁₀-alkenyl, where alkyl and alkenyl radicals may comprise one or more O or S atoms, ester, amide, amino or urethane groups, C₆-C₁₄-aryl or C₇ -C₁₅-alkylaryl;
- X² =: halogen, hydroxy, C₁-C₁₀-alkoxy, C₁-C₆-acyloxy;
- a =: 2, 3 or 4, preferably 3 or 4.

If there are multiple R⁹ or X² residues, they may be different or preferably the same. Preferred alkylaryl groups are -CH₂-CH₂-Ph or a -Ph-CH₃ group with Ph = phenyl.

Particularly preferred are silanes of the Formula (III) in which at least one and preferably all variables have one of the following meanings:
- R⁹ =: C₁-C₁₀-alkyl or C₂-C₈-alkenyl, where alkyl and alkenyl radicals may comprise one or more O atoms or ester groups, phenyl or C₇-C₁₅-alkylaryl;
- X² =: hydroxy, C₁-C₅-alkoxy-, C₁-C₃-acyloxy;
- a =: 3 or 4.

The silanes of Formula (III) can be hydrolytically condensed via the radicals X² to form an inorganic network with Si-O-Si units. If the radicals R⁹ contain radically polymerizable groups, an organic network can also be formed via them.

More preferred are silanes of the Formula (III) where the variables have the following meanings:
- R⁹: methyl, ethyl, n-propyl, i-propyl, n-butyl, s-butyl, n-pentyl, vinyl, a (meth)acryl group (H₂C=C(CH₃ )-COO-), allyl, phenyl or naphthyl, particularly preferred are ethyl, n-propyl, i-propyl and most preferred are methyl or 3-(meth)acryloyloxypropyl;
- X²: methoxy, ethoxy, n-propoxy, i-propoxy, n-butoxy, F, Cl, Br, especially preferably Cl and most preferably ethoxy;
- a: 3 or 4.

According to the invention, silanes of the formula SiX²₄ (a = 4) are particularly preferred, with Si(OCH₃)₄ and Si(OC₂H₅)₄ being even more preferred. Silanes of the formula SiX²₄ give a particularly high network density. They are preferably used in an amount of 40 to 100% by weight, more preferably 50 to 100% by weight and most preferably 60 to 95% by weight, based on the total amount of silanes of Formula (III).

Further preferred are silanes of Formula (III) containing at least one free-radically polymerizable group, 3-methacryloyloxypropyltrimethoxysilane being particularly preferred. The radically polymerizable group enables covalent bonding between the coated fillers and the radically polymerizable matrix during curing of the materials according to the invention. Silanes containing radically polymerizable groups are preferably used in an amount of 0 to 10% by weight, more preferably 0 to 8% by weight and most preferably 1 to 5% by weight, based on the total amount of silanes of the Formula (III).

In addition to the silane(s) of Formula (III), one or more further hydrolytically condensable compounds of the elements Al, Ti, B or P can be used to coat the fillers. Preferred compounds are those of Al, Ti and B. Preferred aluminum compounds are aluminum sec-butylate and aluminum isopropylate. Preferred titanium compounds are Ti(OC₂H₅)₄, Ti(OC₃H₇)₄, Ti(O-iC₃H₇)₄ and Ti(OC₄H₉)₄. Preferred boron compounds are B(OCH₃)₃ and B(OC₂H₅)₃ . By using additional hydrolytically condensable compounds, the properties of the coating, such as hardness and abrasion resistance, can be specifically controlled. One or more additional compounds may be used. These are preferably used in a total amount of 0 to 20% by weight, more preferably 0 to 15% by weight and most preferably 0 to 10% by weight, based on the total amount of silanes of Formula (V). Metal alkoxides, for example of Al or Ti, can also be used in complexed form, for example in the form of the reaction products of Al or Ti alkoxides with (meth)acrylic acid or 2-acetoacetoxyethyl methacrylate. The proportion of silanes of the Formula (III) is preferably 80 to 100% by weight, more preferably 85 to 100% by weight and most preferably 90 to 100% by weight, based on the total amount of hydrolytically condensable compounds.

The silane or silanes of Formula (III) and optionally other hydrolytically condensable compounds are hydrolyzed and condensed in the presence of water, preferably at least the amount of water stoichiometrically required for hydrolysis, and optionally a condensation catalyst and/or a solvent. Polycondensates or heteropolycondensates are formed in this process. Instead of the hydrolytically condensable compounds, precondensates derived therefrom can also be used. Precondensates are reaction products of the alkoxysilanes and/or halosilanes and optionally of the metal alkoxides with water with partial or complete elimination of the alcohols or hydrogen halides. In the case of the alkoxysilanes, silanols are formed as precondensates.

Preferably, the hydrolytic condensation is carried out by dispersing the filler to be coated in the silane(s) of Formula (III) and, if desired, further hydrolytically condensable compounds or, preferably, a solution of the silane(s) and, if desired, further hydrolytically condensable compounds in a suitable solvent and then adding water. The mixture is preferably stirred in this process.

If only silanes are used, the hydrolysis is preferably carried out at room temperature or under slight cooling. Preferably, a hydrolysis or condensation catalyst is added. Polycondensates are obtained in this process. The resulting mixture (dispersion) is preferably stirred for several hours to several days at a temperature of -20 to 150°C, preferably 20 to 110°C. The filler is then separated, e.g. by centrifugation, then optionally washed with a solvent one or more times and then dried. Drying can be carried out at room temperature or at elevated temperature, preferably at 50 to 60°C. Preferably, the filler is dried in a vacuum drying cabinet. Drying may extend over a period of several hours to several days. Preferably, drying is carried out until the weight is constant. After drying, the fillers may be subjected to further heat treatment. This is preferably carried out at a temperature of from 20 to 150°C, more preferably from 30 to 100°C and most preferably from 40 to 80°C. Temperature and treatment time are selected so that any organic groups present in the coating are not destroyed and no glass layer is formed.

If, in addition to the silanes of Formula (V), other hydrolytically condensable compounds are used to coat the filler, the water is preferably added in stages at -20 to 100°C, preferably at 0 to 30°C. In this case, heteropolycondensates are formed. The method of water addition depends primarily on the reactivity of the hydrolytically condensable compounds used. The desired amount of water can be added, for example, in portions or in one portion.

In the preparation of both polycondensates and heteropolycondensates, the water is preferably added in an amount of 0.5 to 10 moles of water per mole of hydrolyzable groups, particularly preferably in an amount of 1 to 10 moles of water per mole of hydrolyzable groups and most preferably in an amount of 1.5 to 5 moles of water per mole of hydrolyzable groups of the silanes of Formula (III) and optional other hydrolytically condensable compounds. The water or portions thereof may be introduced in pure form or as a constituent of other components, e.g. of the catalyst solution.

Preferred solvents for performing the hydrolytic condensation are lower aliphatic alcohols, such as ethanol or i-propanol, lower dialkyl ketones, such as acetone or methyl isobutyl ketone, cyclic ethers, such as tetrahydrofuran or dioxane, aliphatic ethers, such as isopropoxyethanol or isopropoxypropanol, amides, such as dimethylformamide, and mixtures thereof.

Preferred hydrolysis or condensation catalysts are organic or inorganic acids, such as hydrochloric acid, sulfuric acid, p-toluenesulfonic acid, formic acid or acetic acid. Ammonium fluoride and organic bases, in particular amines, such as n-propylamine, diethylamine or triethylamine, and aminosilanes, such as 3-aminopropyltrimethoxysilane, are further preferred.

The condensation time depends on the respective starting components, their proportions, the catalyst used, if any, the reaction temperature. Preferably, the reaction time is in the range of 1 to 96 h, more preferably 2 to 72 h and most preferably 3 to 48 h.

When drying the fillers and evaporating the solvent, the hydrolysis and condensation reaction leads to the formation of a gel film via the formation of sol particles, which further cross-links in the course of drying and forms a dense inorganic network on the filler surface. The properties of the coating, such as strength, flexibility, adhesion, hardness, refractive index and impermeability, can be specifically adjusted by the selection of the silanes and the other hydrolytically condensable components and adapted to the particular application. For example, the functionality of the silanes of Formula (V), which can vary between 4, e.g. in tetraethoxysilane (TEOS), and 2, e.g. in dialkoxysilane, can be used to adjust the degree of crosslink-linking and thus the strength, flexibility and impermeability of the coating. By selecting the organic groups of the silanes, it is possible to specifically influence, for example, the flexibility, polarity, wetting or thickening effect of the coating. Silanes with polymerizable groups, such as in 3-methacryloyloxypropyltrimethoxysilane, allow additional crosslinking of the coating by radical polymerization and covalent incorporation of the fillers into the polymer matrix. Co-condensation with metal alkoxides, such as Ti alkoxide, allows control of the kinetics of the hydrolytic condensation and, for example, an increase in density and hardness as well as adjustment of the refractive index of the coating.

The filler is preferably dispersed in the hydrolysis mixture in an amount of from 1 to 25% by weight, more preferably from 3 to 20% by weight and most preferably from 4 to 15% by weight, based on the total mass of the mixture. According to the invention, particulate fillers are preferred.

Furthermore, so-called inert fillers can also be used as fillers. These are glass fillers whose surface is coated with a diffusion barrier layer, e.g. on a sol-gel basis, or with a polymer layer, e.g. of PVC. Preferred fillers are those described in EP 2 103 296 A1.

Polymer particles, in particular particles of crosslinked organic polymers, are preferred as organic fillers. Polymer particles can be obtained by curing one or more monomers and then grinding the cured mixture, or by suspension polymerization. The mixtures can be cured by light irradiation or thermally. Radically polymerizable monomers and in particular the monomers listed above are preferred as monomers. Dimethacrylates, specifically bis-GMA, UDMA, TMX-UDMA, DCP, D3MA and mixtures thereof, are most particularly preferred.

Polymer particles prepared by grinding exhibit a splintery appearance under the microscope and are therefore also referred to as splinter polymerizates or splinter polymers. Pearl polymerizates obtained by suspension polymerization in contrast are characterized by spherical particles. Splinter polymerizates are preferred as organic fillers.

In addition, so-called composite fillers, i.e. organic-inorganic fillers, are preferred as fillers. These are also referred to as isofillers. These are fragmentary polymers which in turn contain an inorganic filler, preferably pyrogenic SiO₂ and/or ytterbium trifluoride. Preferred are polymers based on dimethacrylates. For the production of isofillers, the inorganic filler(s) is/are incorporated, for example, into a dimethacrylate resin matrix, and the resulting composite paste is subsequently thermally polymerized and then ground.

A composite filler preferred according to the invention can be prepared, for example, by thermally curing a mixture of Bis-GMA (8.80% by weight), UDMA (6.60% by weight), 1,10-decanediol dimethacrylate (5.93% by weight), dibenzoyl peroxide + 2,6-di-tert. butyl-4-methylphenol (together 0.67 wt.%), glass filler (average grain size 0.4 µm; 53.0 wt.%) and YbF₃ (25.0 wt.%) and subsequent grinding of the cured material to the desired grain size. Another composite filler preferred according to the invention is a hot-cured composite filler ground to the desired grain size and prepared from UDMA (23.58 % by weight), 1,10-decanediol dimethacrylate (5.92 % by weight), dibenzoyl peroxide (0.50 % by weight), fumed silica Aerosil OX50 (50.0 % by weight) and YbF₃ (20.0 % by weight). All percentages refer to the total mass of the composite filler.

Unless otherwise stated, all particle sizes are weight-average particle sizes, wherein particle-sizes within the range of from 0.1 µm to 10 µm are measured by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared, and the scattered light thereof is measured in a flow cell. The scattered-light analysis for calculating particle size and particle-size distribution is performed in accordance with the Mie theory according to DIN/ISO 13320: 2020.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably with a Malvern Zetasizer Nano ZS (Malvern Instruments, Malvern UK) with a He-Ne laser with a wavelength of 633 nm, at a scattering angle of 90° at 25° C.

Particle sizes smaller than 0.1 µm can also be determined by means of SEM or TEM spectroscopy. The transmission electron microscopy (TEM) is preferably carried out with a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The light scattering decreases as the particle size decreases, but fillers with a small particle size have a greater thickening action. The fillers are divided according to their particle size into macrofillers and microfillers, wherein fillers with an average particle size of from 0.2 to 10 µm are called macrofillers and fillers with an average particle size of from approx. 5 to 100 nm are called microfillers. Macrofillers are obtained e.g. by grinding e.g. quartz, radiopaque glasses, borosilicate glasses or glass ceramics and usually consist of splintery parts. Microfillers such as mixed oxides can be prepared e.g. by hydrolytic co-condensation of metal alkoxides.

To improve the bond between the filler particles and the crosslinked polymerization matrix, the fillers are preferably surface-modified, particularly preferably by silanization, most particularly preferably by radically polymerizable silanes, in particular with 3-methacryloyloxypropyltrimethoxysilane. For the surface-modification of non-silicate fillers, e.g. of ZrO₂ or TiO₂, functionalized acidic phosphates, such as e.g. 10-methacryloyloxydecyl dihydrogen phosphate can also be used.

The compositions according to the invention preferably contain from 20% to 90% by weight, more preferably from 40% to 85% by weight and most preferably from 50% to 80% by weight of fillers, in each case based on the total mass of the composition.

The compositions according to the invention may also contain further **additives,** in particular stabilizers, such as e.g. polymerization stabilizers, dyes, microbiocidal active ingredients, fluoride-ion-releasing additives such as fluoride salts in particular NaF, ammonium fluoride, or fluorosilanes, optical brighteners, fluorescent agents, plasticizers, and/or UV absorbers.

The compositions according to the invention are particularly suitable as dental materials, in particular as luting materials, filling composites and temporary filling materials.

Compositions which comprise:
(a) 0.2 to 10.0 wt.%, preferably 0.3 to 8.0 wt.% and more preferably 0.5 to 5.0 wt.% of crosslinked acylthiourea polymeric particles according to the invention,
(b) 0.1 to 5.0 wt.%, preferably 0.1 to 4.0 wt.% and more preferably 0.2 to 2.0 wt.% of at least one hydroperoxide compound,
(c) 5 to 80 wt.%, preferably 10 to 60 wt.% and more preferably 15 to 50 wt.% of at least one radically polymerizable monomer,
(d) 20 to 90 wt.%, preferably 40 to 85 wt.% and more preferably 50 to 80 wt.% filler(s),
(e) 0.001 to 5 wt.%, preferably 0.01 to 3 wt.%, additive(s) and
(f) optionally 0.0001 to 0.5 wt.%, preferably 0.0005 to 0.1 wt.% and particularly preferably 0.001 to 0.02 wt.% of at least one transition metal compound
are preferred according to the invention for the use as dental material. The compositions can comprise one or more type of crosslinked acylthiourea polymeric particles, and the weight ranges given above relate to the total mass of these particles.

All quantities herein are relative to the total mass of the composition, unless otherwise stated.

The filling level depends on the desired intended use of the composition. Filling composites preferably have a filler content of from 60 to 85 wt.%.

Compositions comprising crosslinked acylthiourea polymeric particles according to the invention and at least one hydroperoxide are self-curing. They are preferably used in the form of two spatially separated components, i.e. as a **2-component system (2C system).** Oxidizing and reducing agents are incorporated into separate components of the composition. One component, the so-called catalyst paste, contains the hydroperoxide, and optionally a photoinitiator, and the second component, the so-called base paste, contains the acylthiourea oligomer, optionally a photoinitiator and optionally catalytic amounts of a transition metal compound. Polymerization is initiated by mixing the components. Compositions containing both a redox initiator and a photoinitiator are referred to as dual-curing. The pastes are mixed together shortly before use, preferably with a double-push syringe. Base paste and catalyst paste are preferably mixed with each other in a volume ratio of 1:1. The compositions specified above relate to the mixed pastes.

The compositions according to the present invention preferably comprise a **catalyst paste** comprising:
(b) 0.2 to 10.0 wt.%, preferably 0.2 to 8.0 wt.% and more preferably 0.4 to 4.0 wt.% of at least one hydroperoxide compound,
(c) 5 to 80 wt.%, preferably 10 to 60 wt.% and more preferably 15 to 50 wt.% of at least one radically polymerizable monomer,
(d) 20 to 90 wt.%, preferably 40 to 85 wt.% and more preferably 50 to 80 wt.% filler(s), and
(e) 0.001 to 5 wt.%, preferably 0.01 to 3 wt.%, additive(s),

in each case relative to the total mass of the catalyst paste, and
a **base paste** comprising:
   (a) 0.4 to 20.0 wt.%, preferably 0.6 to 16.0 wt.% and more preferably 1.0 to 10.0 wt.% of crosslinked acylthiourea polymeric particles according to the invention,
   (c) 5 to 80 wt.%, preferably 10 to 60 wt.% and more preferably 15 to 50 wt.% of at least one radically polymerizable monomer,
   (d) 20 to 90 wt.%, preferably 40 to 85 wt.% and more preferably 50 to 80 wt.% filler(s),
   (e) 0.001 to 5 wt.%, preferably 0.01 to 3 wt.%, additive(s), and
   (f) 0.0002 to 1.0 wt.%, preferably 0.0010 to 0.2 wt.% and particularly preferably 0.002 to 0.04 wt.% of at least one transition metal compound,
in each case relative to the total mass of the base paste.

The compositions according to the invention are particularly suitable as dental materials for intraoral use by the dentist for the restoration of damaged teeth (therapeutic use), especially for use as temporary materials, filling composites and luting materials.

Two-component composites according to the invention can be used advantageously for the direct filling therapy, e.g., for the treatment of caries. In the case of caries, the dentist first removes the carious tissues, e.g., with a rose-head burr. After the optional application of a dental adhesive, a composite according to the invention is placed in the cavity. Due to the redox polymerization, a high depth of cure can be achieved. According to a preferred embodiment of the invention, the composite material additionally comprises a photoinitiator, and an additional photopolymerization step is performed. In the case of self-adhesive two-component composites, the material can be applied directly to the tooth without the need of a dental adhesive.

The compositions according to the invention can also be used for the fabrication of high-quality temporaries for dental crowns and bridges as well as for temporary inlays, onlays and veneers directly at chairside.

Furthermore, the compositions according to the invention can also be used for the cementation of indirect restorations such as crowns, bridges, inlays, onlays or veneers. In this case, the described compositions are used to mediate a strong bond between the tooth substance and the indirect restoration, e.g., a ceramic restoration or a cured composite. They can be used as luting composites or self-adhesive resin cements.

The invention is explained in more detail below with reference to examples.

### Examples

### Synthesis of a polymerizable acylthiourea methacrylate

### Example 1

### Synthesis of 1-(methacryloyloxy)propan-2-yl 5-oxo-5-thioureidopentanoate TU1

### Step 1: Synthesis of 5-[(1-methacryloyloxypropan-2-yl)oxy]-5-oxopentanoic acid

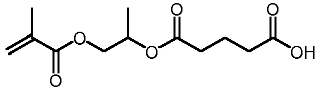

A solution of triethylamine (48.63 g, 0.48 mol), 2-hydroxypropyl methacrylate (69.28 g, 0.48 mol), and 4-dimethylaminopyridine (0.29 g, 2.4 mmol) in dichloromethane (150 mL) was slowly added to a solution of glutaric anhydride (54.83 g, 0.48 mol) in dichloromethane (300 mL) at 0°C. The reaction mixture was stirred at 0°C for 2h and at ambient temperature for 18 h. The reaction mixture was washed with hydrochloric acid (2 *N,* 3× 200 mL), water (2 × 200 mL), and brine (200 mL), dried over anhydrous magnesium sulfate, filtered, and the solvent was evaporated. The residue was dissolved in ethyl acetate (500 mL) and cooled to 0°C. Cold saturated aqueous Na₂CO₃ solution (400 mL) was added while stirring. The phases were separated and the aqueous phase was washed with ethyl acetate (3x 150mL). The aqueous phase was acidified with hydrochloric acid (2N, 480 mL) and extracted with ethyl acetate (4x 100 mL). The combined extracts were dried over anhydrous magnesium sulfate, filtered, and the solvent was evaporated. The residue was dried under reduced pressure, affording 65.56 g (0.25 mol, 53%) of the desired product as a yellowish liquid.

¹H-NMR (CDCl₃, 400 MHz): δ = 6.14 - 6.08 (m, 1H; =CH), 5.62 - 5.55 (m, 1H; =CH), 5.28 - 5.15 (m, 1H; CH), 4.30 - 4.08 (m, 2H; O-CH₂), 2.48 - 2.36 (m, 4H; CH₂), 2.01 - 1.90 (m, 5H; CH₂, CH₃), 1.32 - 1.25 (m, 3H; CH₃).

### Step 2: Synthesis of 1-(methacryloyloxy)propan-2-yl 5-oxo-5-thioureidopentanoate TU1

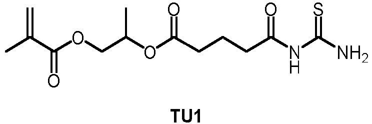

5-[(1-Methacryloyloxypropan-2-yl)oxy]-5-oxopentanoic acid (10.0 g, 38.7 mmol) was dissolved in dichloromethane (100 mL). N,N-Dimethylformamide (28 mg, 0.39 mmol) and thionyl chloride (4.61 g, 38.7 mmol) were added and the reaction mixture was stirred at ambient temperature for 22 h. Thiourea (7.37 g, 96.8 mmol) was added and the suspension was heated to reflux for 24 h. After cooling to RT, the suspension was filtered. The yellow filtrate was washed with water (2x 100 mL), saturated aqueous NaHCO₃ solution (100 mL), and brine (100 ml), dried over anhydrous sodium sulfate, filtered through a pad of silica gel, and the solvent was evaporated. The residue was dissolved in toluene (50 mL). The solution was cooled in an ice bath and stirred until a solid precipitated. *n*-Heptane (25 mL) was added and the suspension was filtered. The residue was washed with *n*-heptane/toluene 1:2 (50 mL) and dried under reduced pressure, affording 7.62 g (24.1 mmol; 62%) of the desired product as a white solid.

¹H-NMR (CDCl₃, 400 MHz): δ = 9.90 (br s, 1H; NH), 9.73 (br s, 1H; NH), 7.71 (br s, 1H; NH), 6.15 - 6.10 (m, 1H; =CH), 5.63 - 5.58 (m, 1H; =CH), 5.29 - 5.17 (m, 1H; CH), 4.32 - 4.10 (m, 2H; O-CH₂), 2.53 - 2.34 (m, 4H; CH₂), 2,05 - 1.88 (m, 5H; CH₂, CH₃), 1.29 (d, *J* = 6.5 Hz, 3H; CH₃).

### Synthesis of thiourea crosslinked polymeric particles

### Example 2

### Synthesis of the ATUG1

1,6-Bis-[2-methacryloyloxyethoxycarbonylamino]-2,4,4-trimethylhexane (UDMA, 0.697 g, 1.49 mmol), TU1 (0.470 g, 1.49 mmol), and isobornyl methacrylate (0.440 g, 1.98 mmol) were dissolved in 2-butanone (10 mL) and the solution was sparged with nitrogen for 15 min. 2-Mercaptoethan-1-ol (58.0 mg, 0.74 mmol) and AIBN (8.2 mg, 0.05 mmol) were added and the reaction mixture was stirred at 77 °C for 2 h. The reaction mixture was dripped into cold diethyl ether (-30 °C, 200 mL). The resulting suspension was stirred for 30 min and filtered. The residue was dried under reduced pressure, affording the desired crosslinked polymeric particles (1.00 g, 62%) as a white solid.

### Example 3

### Synthesis of the ATUG2

UDMA (0.698 g, 1.48 mmol), TU1 (0.470 g, 1.48 mmol), and isobornyl methacrylate (0.440 g, 1.98 mmol) were dissolved in 2-butanone (10 mL) and the solution was sparged with nitrogen for 15 min. 2-Mercaptoethan-1-ol (39.0 mg, 0.50 mmol) and AIBN (8.1 mg, 0.05 mmol) were added and the reaction mixture was stirred at 77 °C for 2 h. The solvent was evaporated, and the residue was washed with methanol (3x 30 mL) and dried, affording the desired crosslinked polymeric particles (1.24 g, 75%) as a pale-yellow solid.

### Example 4

### Synthesis of the ATUG3

Tetraethylene glycol dimethacrylate (1.32 g, 4.00 mmol), TU1 (759 mg, 2.40 mmol), and isobornyl methacrylate (356 mg, 1.60 mmol) were dissolved in 2-butanone (13 mL) and the solution was sparged with nitrogen for 15 min. 2-Mercaptoethan-1-ol (93.8 mg, 1.20 mmol) and AIBN (13.1 mg, 0.08 mmol) were added and the reaction mixture was stirred at 77 °C for 2 h. After cooling to RT, the solution was dripped into diethyl ether (200 mL). The resulting suspension was stirred for 1 h and filtered. The residue was dried under reduced pressure, affording the desired crosslinked polymeric particles (1.47 g, 60%) as a white solid.

### Example 5

### Synthesis of the ATUG4

Methyl methacrylate (0.32 g, 3.20 mmol), TU1 (1.52 g, 4.80 mmol), and tetraethylene glycol dimethacrylate (2.64 g, 8.00 mmol) were dissolved in 2-butanone (25 mL) and the solution was sparged with nitrogen for 15 min. 2-Mercaptoethan-1-ol (188 mg, 2.40 mmol) and AIBN (26.3 mg, 0.16 mmol) were added and the reaction mixture was stirred at 77 °C for 2 h. The solvent was evaporated, and the residue was washed with methanol (4x 30 mL) and dried, affording the desired crosslinked polymeric particles (3.47 g, 74%) as a pale-yellow solid.

### Example 6

### Synthesis of the ATUG5

Butyl methacrylate (0.46 g, 3.20 mmol), TU1 (1.52 g, 4.80 mmol), and tetraethylene glycol dimethacrylate (2.64 g, 8.00 mmol) were dissolved in 2-butanone (25 mL) and the solution was sparged with nitrogen for 15 min. 2-Mercaptoethan-1-ol (188 mg, 2.40 mmol) and AIBN (26.3 mg, 0.16 mmol) were added, and the reaction mixture was stirred at 77 °C for 2 h. The solvent was evaporated, and the residue was washed with methanol (4x 30 mL) and dried, affording the desired crosslinked polymeric particles (3.21 g, 66%) as a pale-yellow solid.

### Example 7

### Preparation of self-cure composites

The catalyst paste C1 listed in Table 1 was prepared by homogenous mixing of the components named in the table. The pastes contained the following monomers: Bis-GMA, bis-[(2-methacryloyloxyethoxycarbonyl)amino]2,2,4(2,4,4)-trimethylhexane (UDMA) and 2-(2-phenylphenoxy)ethyl methacrylate (PEM). Methylhydroquinone (MEHQ) was added as a stabilizer. Cumene hydroperoxide (CHP) was used as oxidant. The barium-aluminium-borosilicate glass filler GM27884 (Schott AG, average particle size 1 µm; BET specific surface area (BET DIN ISO 9277: 2010) 3.9 m²/g; composition (% by weight): Al₂O₃: 10, B₂O₃: 10, BaO: 25 and SiO₂: 55) and a spherical SiO₂-ZrO₂ mixed oxide (Spherosil, Transparent Materials) were used as fillers.

**Table 1: Composition of catalyst paste C1**

| **Component** | **C1 [wt%]** |
|---|---|
| **Bis-GMA** | 11.394 |
| **UDMA** | 15.192 |
| **PEM** | 11.394 |
| **CHP** | 2.00 |
| **MEHQ** | 0.02 |
| **Mixed oxide Spherosil** | 10.00 |
| **Glass filler GM27884** | 50.00 |

The base pastes B1-B6 listed in Table 2 were prepared by homogeneous mixing of the components named in the table. TEMPO (CAS Registry Number 2564-83-2) was used as additional stabilizer. The barium-aluminium-borosilicate glass filler GM27884 (Schott AG, average particle size 1 µm; BET specific surface area (BET DIN ISO 9277: 2010) 3.9 m²/g; composition (% by weight ): Al₂O₃: 10, B₂O₃: 10, BaO: 25 and SiO₂: 55) and a spherical SiO₂-ZrO₂ mixed oxide (Spherosil, Transparent Materials) were used as fillers. Copper(II) acetylacetonate (Cu(acac)₂) was used as transition metal compound. For the comparative paste B6, acetylthiourea (ATU) was used as a reducing agent.

**Table 2: Composition of base pastes B1 - B6**

| **Component** | **B1 [wt%]** | **B2 [wt%]** | **B3 [wt%]** | **B4 [wt%]** | **B5 [wt%]** | **B6*[wt%]** |
|---|---|---|---|---|---|---|
| **Bis-GMA** | 10.7892 | 10.7892 | 10.7892 | 10.7892 | 10.7892 | 11.8092 |
| **UDMA** | 14.3856 | 14.3856 | 14.3856 | 14.3856 | 14.3856 | 15.7456 |
| **PEM** | 10.7892 | 10.7892 | 10.7892 | 10.7892 | 10.7892 | 11.8092 |
| **ATUG1** | 4.00 | - | - | - | - | - |
| **ATUG2** | - | 4.00 | - | - | - | - |
| **ATUG3** | - | - | 4.00 | - | - | - |
| **ATUG4** | - | - | - | 4.00 | - | - |
| **ATUG5** | - | - | - | - | 4.00 | - |
| **ATU** | - | - | - | - | - | 0.60 |
| **Cu(acac)₂** | 0.008 | 0.008 | 0.008 | 0.008 | 0.008 | 0.008 |
| **MEHQ** | 0.02 | 0.02 | 0.02 | 0.02 | 0.02 | 0.02 |
| **TEMPO** | 0.008 | 0.008 | 0.008 | 0.008 | 0.008 | 0.008 |
| **Mixed oxide Spherosil** | 10.00 | 10.00 | 10.00 | 10.00 | 10.00 | 10.00 |
| **Glass filler GM27884** | 50.00 | 50.00 | 50.00 | 50.00 | 50.00 | 50.00 |

| | | | | | | |
|---|---|---|---|---|---|---|
| * comparative example | | | | | | |

### Example 8

### Measurement of the mechanical properties (flexural strength and modulus) of self-cure composites

The self-curing composites listed in Table 3 were prepared by mixing catalyst paste and base paste by hand in a ratio of 1/1 vol/vol. Flexural strength specimens (2 mm x 2 mm x 25 mm) were prepared using a stainless-steel mold. The mold was filled with the cement, then covered with a polyethylene film and placed in an oven at 37°C for 45 min. The specimens were removed from the mold and were stored in water at 37°C for 24 h. The measurement of the flexural strength and modulus was carried out in three-point bending tests (span: 20 mm) according to ISO4049 (Dentistry - Polymer-based filling, restorative and luting materials) with a speed of 0.8 mm min⁻¹ using a Z2.5/TS universal testing machine (Zwick, Germany).

**Table 3: Mechanical properties of self-cure composites (SCC)**

| **Self-cure composite** | **Flexural strength [MPa]** | **Flexural modulus [GPa]** |
|---|---|---|
| **SCC1 (C1 + B1)** | 97.2 ± 10.6 | 7.3 ± 0.4 |
| **SCC2 (C1 + B2)** | 94.0 ± 5.9 | 7.4 ± 0.2 |
| **SCC3 (C1 + B3)** | 90.7 ± 5.6 | 6.7 ± 0.1 |
| **SCC4 (C1 + B4)** | 89.3 ± 7.7 | 6.9 ± 0.2 |
| **SCC5 (C1 + B5)** | 93.7 ± 10.9 | 7.3 ± 0.2 |
| **SCC6 (C1 + B6)*** | 117.5 ± 4.0 | 7.8 ± 0.2 |

| | | |
|---|---|---|
| * comparative example | | |

The data in Table 3 show that the acylthiourea polymer particles according to the invention resulted in composites with excellent mechanical properties. The reference composite **SCC6,** containing ATU as reducing agent, was also evaluated. **SCC6** exhibited the highest flexural strength and modulus values. This composite, however, is too reactive. Indeed, as shown in example 9, its working time is too short for a clinical use.

### Example 9

### Measurement of the working time of self-cure composites

The cements listed in Table 4 were prepared by mixing catalyst paste and base paste by hand in a ratio of 1/1 vol/vol. The working time was determined with an oscillating rheometer (Rheometer MCR 302, Anton Paar) using a plate / plate geometry, at 28.7°C. The storage modulus was recorded as a function of time (frequency = 1 Hz). A graph representing the storage modulus (logarithmic) as a function of time (non-logarithmic) was then used for the determination of the working time. The inflexion point (to be found where the slope of the function reaches its highest value) was identified. A second point on the curve was set as soon as the storage modulus started to rise (end of the stable phase). A straight line was then drawn between the two points. The point on the curve (before the inflexion point) where the tangent line is parallel to this straight line gives the working time.

**Table 4: Working times of self-cure composites (SCC)**

| **Self-cure composites** | **Working time [s]** |
|---|---|
| **SCC1 (C1 + B1)** | 176 ± 11 |
| **SCC2 (C1 + B2)** | 198 ± 9 |
| **SCC3 (C1 + B3)** | 187 ± 11 |
| **SCC4 (C1 + B4)** | 183 ± 6 |
| **SCC5 (C1 + B5)** | 162 ± 6 |
| **SCC6 (C1 + B6)^{*}** | 84 ± 2 |

| | |
|---|---|
| * comparative example | |

Table 4 shows, that the composites according to the invention had good working times.

For the development of self-cure composites, working times of 2 to 4 minutes are usually targeted. SCC6 is however not suitable for a clinical use.

## Claims

1. Crosslinked polymeric particles obtainable by copolymerization of at least one difunctional radically polymerizable monomer and optionally at least one monofunctional radically polymerizable monomer, **characterized in that** the copolymerization is carried out in the presence of a chain transfer agent and of at least one thiourea derivative according to the following Formula (I): 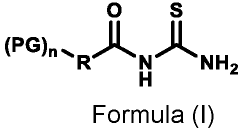 in which the variables have the following meanings:
R is an (n+1)-valent, aromatic, aliphatic, linear or branched C₁-C₅₀ hydrocarbon radical, which can be interrupted by one or more, preferably 1 to 8, particularly preferably 1 to 6, ether, thioether, ester, amide or urethane groups,
PG a radically polymerizable (meth)acrylate, (meth)acrylamide or vinyl group and
n is 1 or 2.

2. The crosslinked polymeric particles according to claim 1, in which the variables have the following meanings:
R an (n+1)-valent, aromatic, aliphatic, linear or branched C₁-C₃₀ hydrocarbon radical, which can be interrupted by one or more, preferably 1 to 6, particularly preferably 1 to 3, ether, ester or urethane groups, preferably an (n+1)-valent, aliphatic, linear or branched C₁-C₂₀ hydrocarbon radical, which can be interrupted by oner or more, preferably 1 to 6, ether, ester or urethane group,
PG a radically polymerizable methacrylate or vinyl group, preferably a radically polymerizable methacrylate group, and
n is 1 or 2.

3. The crosslinked polymeric particles according to any one of claims 1 to 2, which are obtainable by the copolymerization of a monomer mixture comprising:
(1) 10 to 80 mol%, preferably 15 to 60 mol% and more preferably 20 to 50 mol% of the at least one thiourea derivative according to Formula (I),
(2) 10 to 80 mol%, preferably 15 to 70 mol% and more preferably 20 to 60 mol% of the at least one difunctional monomer and
(3) 10 to 80 mol%, preferably 15 to 70 mol% and more preferably 20 to 60 mol% of the at least one monofunctional monomer,
in each case based on the total molar amount of monomers (1) to (3),
and wherein the at least one chain transfer agent is added in an amount at least 10 mol%, preferably 10 to 30 mol%, more preferably 10 to 25 mol% and most preferably 10 to 20 mol%, based on the total molar amount of monomers (1) to (3).

4. The crosslinked polymeric particles according to any one of claims 1 to 3,
- wherein the at least one difunctional monomer is selected from the group consisting of di(meth)acrylates, di(meth)acrylamides, di-N,N-dialkyl(meth)acrylamides and mixtures thereof, and/or
- wherein the at least one monofunctional monomer is selected from the group consisting of (meth)acrylates, (meth)acrylamides, N,N-dialkyl(meth)acrylamides, styrene and derivatives thereof, vinyl acetate, N-vinylpyrrolidone and mixtures thereof, and/or
- wherein the at least one chain transfer agent is selected from the group consisting of mercaptans, disulfides, vinyl ethers, allyl sulfides, allyl sulfones, allyl halides, allyl phosphonates and mixtures thereof.

5. Redox initiator system for the radical polymerization, comprising the crosslinked polymeric particles according to any one of claims 1 to 4 and at least one hydroperoxide.

6. Radically polymerizable composition, comprising the crosslinked polymeric particles according to any one of claims 1 to 4, at least one hydroperoxide and at least one radically polymerizable monomer.

7. The redox initiator system of claim 5 or the radically polymerizable composition according to claim 6, which comprises a hydroperoxide of the formula R⁴-(OOH)_{y}, in which R⁴ is an aliphatic or aromatic hydrocarbon radical and y is 1 or 2, preferably a hydroperoxide that is selected from the group consisting of t-amyl hydroperoxide, 1,1,3,3-tetramethylbutyl hydroperoxide, t-butyl hydroperoxide, t-hexyl hydroperoxide 2,5-dimethyl-2,5-di(hydroperoxy)hexane, diisopropylbenzene monohydroperoxide, paramenthane hydroperoxide, p-isopropyl-cumene hydroperoxide, cumene hydroperoxide (CHP) and or a mixture thereof, and/or
a hydroperoxide according to the following Formula (II) 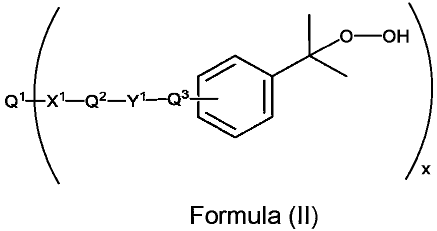
in which the variables have the following meanings:
Q¹ a x-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR⁵, -Cl and -Br, wherein R⁵ is an aliphatic, linear or branched C₁-C₁₀ hydrocarbon radical,
X¹, Y¹ independently of each other are in each case absent, -O-, -COO-; -CONR⁶-, or -O-CO-NR⁷-, wherein R⁶ and R⁷ independently of each other represent H or a C₁-C₅ alkyl radical, preferably H, methyl and/or ethyl, particularly preferably H, and wherein X¹ and Y¹ are preferably not absent at the same time, and wherein X¹ and/or Y¹ is absent if Q² is absent,
Q² is absent, an aliphatic, linear or branched C₁-C₁₄ alkylene radical, which can be interrupted by S and/or O atoms and which can be unsubstituted or substituted by -OH, -OR⁸, -CI and/or -Br, wherein R⁸ is an aliphatic, linear or branched C₁-C₁₀ hydrocarbon radical,
Q³ a C₁-C₃ alkylene group or is absent, preferably -CH₂- or is absent,
x 1, 2, 3 or 4, and wherein
the substitution on the aromatic ring takes place in position 2, 3 or 4, relative to the cumene hydroperoxide group.

8. The redox initiator system according to any one of claims 5 or 7 or the radically polymerizable composition according to claim 6 or 7 which additionally comprises a transition metal compound, preferably a compound of a transition metal which has at least two stable oxidation states, preferably a compound of copper, cobalt, nickel, vanadium, manganese or a mixture thereof.

9. The radically polymerizable composition according to any one of claims 6 to 8, wherein the at least one radically polymerizable monomer is selected from multifunctional (meth)acrylates or mixtures of mono- and multifunctional (meth)acrylates.

10. The radically polymerizable composition according to claim 9, wherein the at least one multifunctional (meth)acrylate is selected from the group consisting of bisphenol A dimethacrylate, bis-GMA (an addition product of methacrylic acid and bisphenol A diglycidyl ether), ethoxylated or propoxylated bisphenol A dimethacrylate, such as the bisphenol A dimethacrylate SR-348c (Sartomer) with 3 ethoxy groups or 2,2-bis[4-(2-methacryloyloxypropoxy)phenyl]propane, UDMA (an addition product of 2-hydroxyethyl methacrylate and 2,2,4-trimethylhexamethylene-1,6-diisocyanate), 2-{[(2-(methacryloyloxy)ethoxy)carbonyl]-amino} ethyl methacrylate, tetramethylxylylene diurethane ethylene glycol di(meth)acrylate tetramethylxylylene diurethane-2-methylethylene glycoldi(meth)acrylate (V380), di-, tri- or tetraethylene glycol dimethacrylate, trimethylol propanetrimethacrylate, pentaerythritol tetramethacrylate and glycerol di- and trimethacrylate, 1,4-butanediol dimethacrylate, 1,10-decanediol dimethacrylate (D3MA), bis(methacryloyloxymethyl)tricyclo-[5.2.1.0^{2,6}]decane (DCP), polyethylene glycol or polypropylene glycol dimethacrylates, such as polyethylene glycol 200-dimethacrylate or polyethylene glycol 400-dimethacrylate (PEG-200- or PEG-400-DMA), 1,12-dodecanediol dimethacrylate and mixtures thereof, and/or
wherein the at least one monofunctional monomer is selected from the group consisting of benzyl and furfuryl methacrylate, 2-phenoxyethyl methacrylate, 2-(o-biphenyloxy)ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]-ethyl methacrylate, 1-phenoxypropan-2-yl methacrylate and 2-(p-cumylphenoxy)-ethyl methacrylate, tricyclodecane methacrylate, tricyclodecane methyl methacrylate and/or 2-(p-cumylphenoxy)ethyl methacrylate, cumylphenoxyethylene glycol methacrylate (CMP-1E) and mixtures thereof.

11. The radically polymerizable composition according to any one of claims 6 to 10, which additionally comprises at least one acid-group-containing radically polymerizable monomer, preferably a polymerizable carboxylic acid, phosphonic acid, phosphoric acid ester or an anhydride of these substances.

12. The radically polymerizable composition according to any one of claims 6 to 11, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, a glass powder, such as quartz, glass ceramic, borosilicate or radiopaque glass powder, preferably a barium or strontium aluminium silicate glass powder or fluoroaluminosilicate glass powder, a radiopaque filler, such as ytterbium trifluoride, tantalum(V) oxide, barium sulfate, a mixed oxide of SiO₂ with ytterbium(III) oxide or tantalum(V) oxide, a ground polymer, a ground composite filler or a pearl polymer.

13. The radically polymerizable composition according to any one of claims 6 to 12, which comprises
(a) 0.2 to 10.0 wt.%, preferably 0.3 to 8.0 wt.% and more preferably 0.5 to 5.0 wt.% of crosslinked polymeric particles according to any one of claims 1 to 4,
(b) 0.1 to 5.0 wt.%, preferably 0.1 to 4.0 wt.% and more preferably 0.2 to 2.0 wt.% of at least one hydroperoxide,
(c) 5 to 80 wt.%, preferably 10 to 60 wt.% and more preferably 15 to 50 wt.% of at least one radically polymerizable monomer,
(d) 20 to 90 wt.%, preferably 40 to 85 wt.% and more preferably 50 to 80 wt.% filler(s),
(e) 0.001 to 5 wt.%, preferably 0.01 to 3 wt.%, additive(s) and
(f) 0.0001 to 0.5 wt.%, preferably 0.0005 to 0.1 wt.% and particularly preferably 0.001 to 0.02 wt.% of at least one transition metal compound,
in each case relative to the total mass of the material.

14. The radically polymerizable composition according to any one of claims 6 to 13, for therapeutic use in the restoration of damaged teeth, preferably as a dental cement, temporary material, filling composite or luting material.

15. Non-therapeutic use of the radically polymerizable composition according to one of claims 6 to 13, for the production or repair of dental restorations, such as prostheses, artificial teeth, inlays, onlays, crowns, bridges and complete dentures.

## Patentansprüche

1. Vernetzte Polymerpartikel, erhältlich durch Copolymerisation mindestens eines difunktionellen radikalisch polymerisierbaren Monomers und gegebenenfalls mindestens eines monofunktionellen radikalisch polymerisierbaren Monomers, **dadurch gekennzeichnet, dass** die Copolymerisation in Gegenwart eines Kettenübertragungsreagenzes und mindestens eines Thioharnstoffderivats gemäß der folgenden Formel (I) durchgeführt wird: 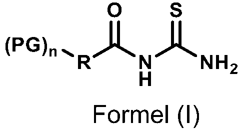 in der die Variablen die folgenden Bedeutungen haben:
R ist ein (n+1)-wertiger, aromatischer, aliphatischer, linearer oder verzweigter C₁-C₅₀-Kohlenwasserstoffrest, der durch eine oder mehrere, vorzugsweise 1 bis 8, besonders bevorzugt 1 bis 6, Ether-, Thioether-, Ester-, Amid- oder Urethangruppen unterbrochen sein kann,
PG ist eine radikalisch polymerisierbare (Meth)acrylat-, (Meth)acrylamid- oder Vinylgruppe und
n ist 1 oder 2.

2. Vernetzte Polymerpartikel gemäß Anspruch 1, wobei die Variablen die folgenden Bedeutungen haben:
R ist ein (n+1)-wertiger, aromatischer, aliphatischer, linearer oder verzweigter C₁-C₃₀-Kohlenwasserstoffrest, der durch eine oder mehrere, vorzugsweise 1 bis 6, besonders bevorzugt 1 bis 3, Ether-, Ester- oder Urethangruppen unterbrochen sein kann, vorzugsweise ein (n+1)-wertiger, aliphatischer, linearer oder verzweigter C₁-C₂₀ Kohlenwasserstoffrest, der durch eine oder mehrere, vorzugsweise 1 bis 6, Ether-, Ester- oder Urethangruppen unterbrochen sein kann,
PG ist eine radikalisch polymerisierbare Methacrylat- oder Vinylgruppe, vorzugsweise eine radikalisch polymerisierbare Methacrylatgruppe, und
n ist 1 oder 2.

3. Vernetzte Polymerpartikel gemäß einem der Ansprüche 1 bis 2, die durch Copolymerisation einer Monomermischung erhältlich sind, die
(1) 10 bis 80 Mol-%, vorzugsweise 15 bis 60 Mol-% und besonders bevorzugt 20 bis 50 Mol-% des mindestens einen Thioharnstoffderivats gemäß Formel (I),
(2) 10 bis 80 Mol-%, vorzugsweise 15 bis 70 Mol-% und besonders bevorzugt 20 bis 60 Mol-% des mindestens einen difunktionellen Monomers und
(3) 10 bis 80 Mol-%, vorzugsweise 15 bis 70 Mol-% und besonders bevorzugt 20 bis 60 Mol-% des mindestens einen monofunktionellen Monomers enthält,
jeweils bezogen auf die Gesamtmolmenge der Monomere (1) bis (3),
und wobei das mindestens eine Kettenübertragungsreagenz in einer Menge von mindestens 10 Mol-%, vorzugsweise 10 bis 30 Mol-%, besonders bevorzugt 10 bis 25 Mol-% und am meisten bevorzugt 10 bis 20 Mol-% zugegeben wird, bezogen auf die Gesamtmolmenge der Monomere (1) bis (3).

4. Vernetzte Polymerpartikel gemäß einem der Ansprüche 1 bis 3,
- wobei das mindestens eine difunktionelle Monomer aus der Gruppe ausgewählt ist, die aus Di(meth)acrylaten, Di(meth)acrylamiden, Di-N,N-dialkyl(meth)acrylamiden und Mischungen davon besteht, und/oder
- wobei das mindestens eine monofunktionelle Monomer aus der Gruppe ausgewählt ist, die aus (Meth)acrylaten, (Meth)acrylamiden, N,N-Dialkyl(meth)acrylamiden, Styrol und Derivaten davon, Vinylacetat, N-Vinylpyrrolidon und Mischungen davon besteht, und/oder
- wobei das mindestens eine Kettenübertragungsreagenz aus der Gruppe ausgewählt ist, die aus Mercaptanen, Disulfiden, Vinylethern, Allylsulfiden, Allylsulfonen, Allylhalogeniden, Allylphosphonaten und Mischungen davon besteht.

5. Redox-Initiatorsystem für die radikalische Polymerisation, das vernetzte Polymerpartikel gemäß einem der Ansprüche 1 bis 4 und mindestens ein Hydroperoxid enthält.

6. Radikalisch polymerisierbare Zusammensetzung, die vernetzte Polymerpartikel gemäß einem der Ansprüche 1 bis 4, mindestens ein Hydroperoxid und mindestens ein radikalisch polymerisierbares Monomer enthält.

7. Redox-Initiatorsystem nach Anspruch 5 oder radikalisch polymerisierbare Zusammensetzung nach Anspruch 6, das oder die ein Hydroperoxid der Formel R⁴-(OOH)_{y}, worin R⁴ ein aliphatischer oder aromatischer Kohlenwasserstoffrest und y 1 oder 2 ist, vorzugsweise ein Hydroperoxid, das aus der Gruppe ausgewählt ist, die aus t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, para-Menthanhydroperoxid, p-Isopropylcumolhydroperoxid, Cumolhydroperoxid (CHP) und/oder einer Mischung davon ausgewählt ist, und/oder
ein Hydroperoxid gemäß der folgenden Formel (II) enthält, 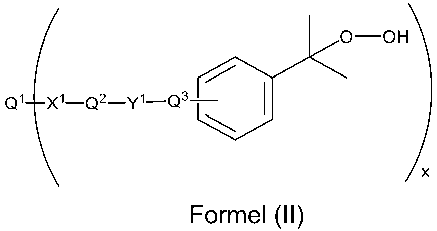
in der die Variablen folgende Bedeutung haben:
Q¹ ist ein x-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten substituiert sein kann, der oder die vorzugsweise aus -OH, -OR⁵, -CI und -Br ausgewählt sind, wobei R⁵ ein aliphatischer, linearer oder verzweigter C₁-C₁₀ Kohlenwasserstoffrest ist,
X¹, Y¹ sind unabhängig voneinander jeweils abwesend, -O-, -COO-; -CONR⁶- oder -O-CO-NR⁷-, wobei R⁶ und R⁷ unabhängig voneinander jeweils H oder ein C₁-C₅-Alkylrest, vorzugsweise H, Methyl und/oder Ethyl, besonders bevorzugt H sind, und wobei X¹ und Y¹ vorzugsweise nicht gleichzeitig fehlen, und wobei X¹ und/oder Y¹ fehlt, wenn Q² fehlt,
Q² fehlt oder ist ein aliphatischer, linearer oder verzweigter C₁ -C₁₄ Alkylenrest, der durch S- und/oder O-Atome unterbrochen sein kann und der unsubstituiert oder durch -OH, -OR⁸, -Cl und/oder -Br substituiert sein kann, wobei R⁸ ein aliphatischer, linearer oder verzweigter C₁-C₁₀-Kohlenwasserstoffrest ist,
Q³ ist eine C₁ -C₃ Alkylengruppe oder fehlt, vorzugsweise -CH₂ - oder fehlt,
x ist 1, 2, 3 oder 4 ist, und wobei
der aromatische Ring in Position 2, 3 oder 4 relativ zur Cumolhydroperoxidgruppe substituiert ist.

8. Redox-Initiatorsystem gemäß einem der Ansprüche 5 oder 7 oder radikalisch polymerisierbare Zusammensetzung gemäß Anspruche 6 oder 7, das oder die zusätzlich eine Übergangsmetallverbindung enthält, vorzugsweise eine Verbindung eines Übergangsmetalls, das mindestens zwei stabile Oxidationsstufen aufweist, vorzugsweise eine Verbindung von Kupfer, Kobalt, Nickel, Vanadium, Mangan oder einer Mischung davon.

9. Radikalisch polymerisierbare Zusammensetzung gemäß einem der Ansprüche 6 bis 8, wobei das mindestens eine radikalisch polymerisierbare Monomer aus multifunktionellen (Meth)acrylaten oder Mischungen aus mono- und multifunktionellen (Meth)acrylaten ausgewählt ist.

10. Radikalisch polymerisierbare Zusammensetzung gemäß Anspruch 9, wobei das mindestens eine multifunktionelle (Meth)acrylat aus der Gruppe ausgewählt ist, die aus Bisphenol-A-dimethacrylat, Bis-GMA (ein Additionsprodukt von Methacrylsäure und Bisphenol-A-diglycidylether), ethoxyliertem oder propoxyliertem Bisphenol-A-dimethacrylat, wie dem Bisphenol-A-dimethacrylat SR-348c (Sartomer) mit 3 Ethoxygruppen oder 2,2-Bis[4-(2-methacryloyloxypropoxy)phenyl]propan, UDMA (ein Additionsprodukt von 2-Hydroxyethylmethacrylat und 2,2,4-Trimethylhexamethylen-1,6-diisocyanat), 2-{[(2-(Methacryloyloxy)ethoxy)carbonyl]amino}ethylmethacrylat, Tetramethylxylylendiurethanethylenglycoldi(meth)acrylat, Tetramethylxylylendiurethan-2-methylethylenglykoldi(meth)acrylat (V380), Di-, Tri- oder Tetraethylenglykoldimethacrylat, Trimethylolpropantrimethacrylat, Pentaerythritoltetramethacrylat und Glyceroldi- und -trimethacrylat, 1,4-Butandioldimethacrylat, 1,10-Decandioldimethacrylat (D3MA), Bis(methacryloyloxymethyl)tricyclo-[5.2.1.0^{2,6}]decan (DCP), Polyethylenglykol- oder Polypropylenglykoldimethacrylate, wie Polyethylenglykol-200-dimethacrylat oder Polyethylenglykol-400-dimethacrylat (PEG-200- oder PEG-400-DMA), 1,12-Dodecandioldimethacrylat und Mischungen davon besteht, und/oder wobei das mindestens eine monofunktionelle Monomer aus der Gruppe ausgewählt ist, die aus Benzyl- und Furfurylmethacrylat, 2-Phenoxyethylmethacrylat, 2-(o-Biphenyloxy)ethylmethacrylat, 2-Hydroxy-3-phenoxypropylmethacrylat, Phenethylmethacrylat, 2-[(Benzyloxycarbonyl)-amino]-ethylmethacrylat, 2-[(Benzylcarbamoyl)oxy]-ethylmethacrylat, 1-Phenoxypropan-2-yl-methacrylat und 2-(p-Cumylphenoxy)ethylmethacrylat, Tricyclodecanmethacrylat, Tricyclodecanmethylmethacrylat und/oder 2-(p-Cumylphenoxy)ethylmethacrylat, Cumylphenoxyethylenglycolmethacrylat (CMP-1E) und Mischungen davon besteht.

11. Radikalisch polymerisierbare Zusammensetzung gemäß einem der Ansprüche 6 bis 10, die zusätzlich mindestens ein säuregruppenhaltiges radikalisch polymerisierbares Monomer enthält, vorzugsweise eine polymerisierbare Carbonsäure, Phosphonsäure, einen polymerisierbaren Phosphorsäureester oder ein Anhydrid dieser Substanzen.

12. Radikalisch polymerisierbare Zusammensetzung gemäß einem der Ansprüche 6 bis 11, die zusätzlich mindestens einen organischen oder anorganischen Füllstoff enthält, vorzugsweise ein Oxid, wie SiO₂, ZrO₂ und TiO₂ oder ein Mischoxid von SiO₂, ZrO₂, ZnO und/oder TiO₂, einen nanopartikulären oder mikrofeinen Füllstoff, wie pyrogene Kieselsäure oder gefällte Kieselsäure, ein Glaspulver, wie Quarz-, Glaskeramik-, Borosilikat- oder röntgenopakes Glaspulver, vorzugsweise ein Barium- oder Strontium-Aluminiumsilikat-Glaspulver oder Fluoraluminiumsilikat-Glaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Polymer, einen gemahlenen Kompositfüllstoff oder ein Perlpolymer.

13. Radikalisch polymerisierbare Zusammensetzung gemäß einem der Ansprüche 6 bis 12, die
(a) 0,2 bis 10,0 Gew.-%, vorzugsweise 0,3 bis 8,0 Gew.-% und besonders bevorzugt 0,5 bis 5,0 Gew.-% vernetzte Thioharnstoff-Polymerpartikel gemäß einem der Ansprüche 1 bis 4,,
(b) 0,1 bis 5,0 Gew.-%, vorzugsweise 0,1 bis 4,0 Gew.-% und besonders bevorzugt 0,2 bis 2,0 Gew.-% mindestens eines Hydroperoxids,
(c) 5 bis 80 Gew.-%, vorzugsweise 10 bis 60 Gew.-% und besonders bevorzugt 15 bis 50 Gew.-% mindestens eines radikalisch polymerisierbaren Monomers,
(d) 20 bis 90 Gew.-%, vorzugsweise 40 bis 85 Gew.-% und besonders bevorzugt 50 bis 80 Gew.-% Füllstoff(e),
(e) 0,001 bis 5 Gew.-%, vorzugsweise 0,01 bis 3 Gew.-%, Additiv(e) und
(f) 0,0001 bis 0,5 Gew.-%, vorzugsweise 0,0005 bis 0,1 Gew.-% und besonders bevorzugt 0,001 bis 0,02 Gew.-% mindestens einer Übergangsmetallverbindung, enthält,
jeweils bezogen auf die Gesamtmasse der Zusammensetzung.

14. Radikalisch polymerisierbare Zusammensetzung gemäß einem der Ansprüche 6 bis 13 zur therapeutischen Verwendung bei der Wiederherstellung beschädigter Zähne, vorzugsweise als Zahnzement, provisorisches Material, Füllungskomposit oder Befestigungsmaterial.

15. Nicht-therapeutische Verwendung der radikalisch polymerisierbaren Zusammensetzung gemäß einem der Ansprüche 6 bis 13 zur Herstellung oder Reparatur von Zahnrestaurationen, wie Prothesen, künstlichen Zähnen, Inlays, Onlays, Kronen, Brücken und Vollprothesen.

## Revendications

1. Particules polymères réticulées pouvant être obtenues par copolymérisation d'au moins un monomère difonctionnel polymérisable par voie radicalaire et en option d'au moins un monomère monofonctionnel polymérisable par voie radicalaire, **caractérisées en ce que** la copolymérisation est effectuée en présence d'un agent de transfert de chaîne et d'au moins un dérivé de thiourée selon la formule (I) suivante : 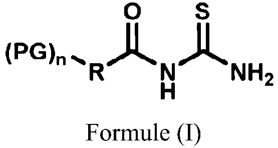 dans laquelle les variables ont les significations suivantes :
R est un radical hydrocarboné en C₁-C₅₀ (n+1)-valent, aromatique, aliphatique, linéaire ou ramifié, qui peut être interrompu par un ou plusieurs, de préférence 1 à 8, de façon particulièrement préférée 1 à 6, groupes éther, thioéther, ester, amido ou uréthane,
PG est un groupe (méth)acrylate, (méth)acrylamido ou vinyle, polymérisable par voie radicalaire et
n est 1 ou 2.

2. Particules polymères réticulées selon la revendication 1, dans lesquelles les variables ont les significations suivantes :
R est un radical hydrocarboné en C₁-C₃₀ (n+1)-valent, aromatique, aliphatique, linéaire ou ramifié, qui peut être interrompu par un ou plusieurs, de préférence 1 à 6, de façon particulièrement préférée 1 à 3 groupes éther, ester ou uréthane, de préférence un radical hydrocarboné en C₁-C₂₀ (n+1)-valent, aliphatique, linéaire ou ramifié, qui peut être interrompu par un ou plusieurs, de préférence 1 à 6, groupes éther, ester ou uréthane,
PG est un groupe méthacrylate ou vinyle, polymérisable par voie radicalaire, de préférence un groupe méthacrylate polymérisable par voie radicalaire et
n est 1 ou 2.

3. Particules polymères réticulées selon l'une quelconque des revendications 1 et 2, qui peuvent être obtenues par la copolymérisation d'un mélange de monomères comprenant :
(1) 10 à 80 % en moles, de préférence 15 à 60 % en moles et plus préférablement 20 à 50 % en moles dudit au moins un dérivé de thiourée selon la formule (I),
(2) 10 à 80 % en moles, de préférence 15 à 70 % en moles et plus préférablement 20 à 60 % en moles dudit au moins un monomère difonctionnel et
(3) 10 à 80 % en moles, de préférence 15 à 70 % en moles et plus préférablement 20 à 60 % en moles dudit au moins un monomère monofonctionnel,
dans chaque cas par rapport à la quantité molaire totale de monomères (1) à (3).
et ledit au moins un agent de transfert de chaîne étant ajouté en une quantité d'au moins 10 % en moles, de préférence 10 à 30 % en moles, plus préférablement 10 à 25 % en moles et le plus préférablement 10 à 20 % en moles, par rapport à la quantité molaire totale de monomères (1) à (3).

4. Particules polymères réticulées selon l'une quelconque des revendications 1 à 3,
- dans lesquelles ledit au moins un monomère difonctionnel est choisi dans l'ensemble constitué par les di(méth)acrylates, di(méth)acrylamides, di-N,N-dialkyl(méth)acrylamides et des mélanges de ceux-ci, et/ou
- dans lesquelles ledit au moins un monomère monofonctionnel est choisi dans l'ensemble constitué par les (méth)acrylates, (méth)acrylamides, N,N-dialkyl(méth)acrylamides, le styrène et ses dérivés, l'acétate de vinyle, la N-vinylpyrrolidone et des mélanges de ceux-ci, et/ou
- dans lesquelles ledit au moins un agent de transfert de chaîne est choisi dans l'ensemble constitué par les mercaptans, disulfures, éthers vinyliques, sulfures d'allyle, allylsulfones, halogénures d'allyle, phosphonates d'allyle et des mélanges de ceux-ci.

5. Système amorceur redox pour la polymérisation radicalaire, comprenant les particules polymères réticulées selon l'une quelconque des revendications 1 à 4 et au moins un hydroperoxyde.

6. Composition polymérisable par voie radicalaire, comprenant les particules polymères réticulées selon l'une quelconque des revendications 1 à 4, au moins un hydroperoxyde et au moins un monomère polymérisable par voie radicalaire.

7. Système amorceur redox selon la revendication 5 ou composition polymérisable par voie radicalaire selon la revendication 6, qui comprend un hydroperoxyde de formule R⁴-(OOH)_{y}, dans laquelle R⁴ est un radical hydrocarboné aliphatique ou aromatique et y est 1 ou 2, de préférence un hydroperoxyde qui est choisi dans l'ensemble constitué par l'hydroperoxyde de tert-amyle, l'hydroperoxyde de 1,1,3,3-tetraméthylbutyle, l'hydroperoxyde de tert-butyle, l'hydroperoxyde de tert-hexyle, le 2,5-diméthyl-2,5-di(hydroperoxy)hexane, le monohydroperoxyde de diisopropylbenzène, l'hydroperoxyde de paramenthane, l'hydroperoxyde de p-isopropylcumène, l'hydroperoxyde de cumène (CHP) et/ou un mélange de ceux-ci, et/ou
un hydroperoxyde selon la formule (II) suivante, 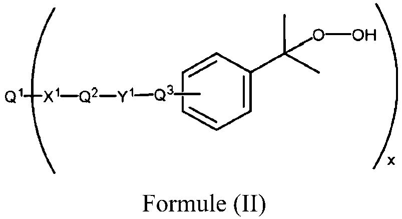
dans laquelle les variables ont les significations suivantes :
Q¹ représente un radical hydrocarboné en C₁-C₁₄ x-valent, aromatique, aliphatique, linéaire ou ramifié, qui peut être interrompu par un ou plusieurs atome(s) d'oxygène et/ou de soufre et qui peut être non substitué ou porteur d'un ou de plusieurs substituant(s) qui est/sont choisi(s) de préférence parmi -OH, -OR⁵, -Cl et -Br, R⁵ étant un radical hydrocarboné en C₁-C₁₀ aliphatique, linéaire ou ramifié,
X¹, Y¹ sont chacun, indépendamment l'un de l'autre, dans chaque cas absents ou -O-, -COO- ; -CONR⁶-, ou -O-CO-NR⁷-, R⁶ et R⁷ représentant indépendamment l'un de l'autre un atome d'hydrogène ou un radical alkyle en C₁-C₅, de préférence un atome d'hydrogène, un groupe méthyle et/ou un groupe éthyle, de façon particulièrement préférée un atome d'hydrogène, et X¹ et Y¹ n'étant de préférence pas absents simultanément, et X¹ et/ou Y¹ étant absent(s) si Q² est absent,
Q² est absent ou est un radical alkylène en C₁-C₁₄ aliphatique, linéaire ou ramifié, qui peut être interrompu par des atomes d'oxygène et/ou de soufre et qui peut être non substitué ou substitué par -OH, -OR⁸, -Cl et/ou -Br, R⁸ étant un radical hydrocarboné en C₁-C₁₀ aliphatique, linéaire ou ramifié,
Q³ est un groupe alkylène en C₁-C₃ ou est absent, de préférence est un groupe -CH₂- ou est absent,
x vaut 1, 2, 3 ou 4, et
la substitution sur le cycle aromatique s'effectuant en position 2, 3 ou 4, par rapport au groupe hydroperoxyde de cumène.

8. Système amorceur redox selon l'une quelconque des revendications 5 et 7 ou composition polymérisable par voie radicalaire selon la revendication 6 ou 7, qui comprend en outre un composé de métal de transition, de préférence un composé d'un métal de transition qui a au moins deux états d'oxydation stables, de préférence un composé de cuivre, cobalt, nickel, vanadium, manganèse ou un mélange de tels composés.

9. Composition polymérisable par voie radicalaire selon l'une quelconque des revendications 6 à 8, dans laquelle ledit au moins un monomère polymérisable par voie radicalaire est choisi parmi les (méth)acrylates multifonctionnels ou des mélanges de (méth)acrylates mono- et multifonctionnels.

10. Composition polymérisable par voie radicalaire selon la revendication 9, dans laquelle ledit au moins un (méth)acrylate multifonctionnel est choisi dans l'ensemble constitué par le diméthacrylate de bisphénol A, le bis-GMA (un produit d'addition d'acide méthacrylique et d'éther diglycidylique de bisphénol A), un diméthacrylate de bisphénol A éthoxylé ou propoxylé, tel que le diméthacrylate de bisphénol A SR-348c (Sartomer) comportant 3 groupes éthoxy ou 2,2-bis[4-(2-méthacryloyloxypropoxy)phényl]propane, l'UDMA (un produit d'addition de méthacrylate de 2-hydroxyéthyle et de 1,6-diisocyanate de 2,2,4-triméthylhexaméthylène), le méthacrylate de 2-{[(2-(méthacryloyloxy)éthoxy)-carbonyl]-amino}éthyle, le di(méth)acrylate de tétraméthylxylylène diuréthane éthylèneglycol, le di(méth)acrylate de tétraméthylxylylène diuréthane-2-méthyléthylèneglycol (V380), le diméthacrylate de di-, tri- ou tétraéthylèneglycol, le triméthacrylate de triméthylolpropane, le tétraméthacrylate de pentaérythritol et le di- et le triméthacrylate de glycérol, le diméthacrylate de 1,4-butanediol, le diméthacrylate de 1,10-decanediol (D3MA), le bis(méthacryloyloxyméthyl)-tricyclo-[5.2.1.02,6]décane (DCP), les diméthacrylates de polyéthylèneglycol ou polypropylèneglycol, tels que le diméthacrylate de polyéthylèneglycol 200 ou le diméthacrylate de polyéthylèneglycol 400 (PEG-200- ou PEG-400-DMA), le diméthacrylate de 1,12-dodécanediol et des mélanges de ceux-ci, et/ou
dans laquelle ledit au moins un monomère monofonctionnel est choisi dans l'ensemble constitué par le méthacrylate de benzyle et de furfuryle, le méthacrylate de 2-phénoxyéthyle, le méthacrylate de 2-(o-biphényloxy)éthyle, le méthacrylate de 2-hydroxy-3-phénoxypropyle, le méthacrylate de phénéthyle, le méthacrylate de 2-[(benzyloxycarbonyl)-amino]-éthyle, le méthacrylate de 2-[(benzylcarbamoyl)-oxy]-éthyle, le méthacrylate de 1-phénoxypropan-2-yle et le méthacrylate de 2-(p-cumylphénoxy)-éthyle, le méthacrylate de tricyclodécane, le méthacrylate de tricyclodécaneméthyle et/ou le méthacrylate de 2-(p-cumylphénoxy)éthyle, le méthacrylate de cumylphénoxyéthylèneglycol (CMP-1E) et des mélanges de ceux-ci.

11. Composition polymérisable par voie radicalaire selon l'une quelconque des revendications 6 à 10, qui comprend en outre au moins un monomère polymérisable par voie radicalaire contenant des groupes acides, de préférence un acide carboxylique, acide phosphonique, ester d'acide phosphorique, polymérisable, ou un anhydride de ces substances.

12. Composition polymérisable par voie radicalaire selon l'une quelconque des revendications 6 à 11, qui comprend en outre au moins une charge organique ou inorganique, de préférence un oxyde, tel que SiO₂, ZrO₂ et TiO₂ ou un oxyde mixte à base de SiO₂, ZrO₂, ZnO et/ou TiO₂, une charge microfine ou nanoparticulaire, telle que la silice pyrogénée ou la silice précipitée, une poudre de verre, telle que la poudre de quartz, la poudre de vitrocéramique, la poudre de borosilicate ou la poudre de verre opaque aux rayons X, de préférence la poudre de verre d'aluminosilicate de baryum ou strontium ou la poudre de verre de fluoroaluminosilicate, une charge opaque aux rayons X, telle que le trifluorure d'ytterbium, l'oxyde de tantale-(V), le sulfate de baryum, un oxyde mixte de SiO₂ avec l'oxyde d'ytterbium-(III) ou l'oxyde de tantale-(V), un polymère broyé, une charge composite broyée ou un polymère en perles.

13. Composition polymérisable par voie radicalaire selon l'une quelconque des revendications 6 et 8 à 12
qui comprend
(a) 0,2 à 10,0 % en poids, de préférence 0,3 à 8,0 % en poids et plus préférablement 0,5 à 5,0 % en poids de particules polymères réticulées selon l'une quelconque des revendications 1 à 4,
(b) 0,1 à 5,0 % en poids, de préférence 0,1 à 4,0 % en poids et plus préférablement 0,2 à 2,0 % en poids d'au moins un hydroperoxyde,
(c) 5 à 80,0 % en poids, de préférence 10 à 60 % en poids et plus préférablement 15 à 50 % en poids d'au moins un monomère polymérisable par voie radicalaire,
(d) 20 à 90 % en poids, de préférence 40 à 85 % en poids et plus préférablement 50 à 80 % en poids de charge(s),
(e) 0,001 à 5 % en poids, de préférence 0,01 à 3 % en poids d'additif(s) et
(f) 0,0001 à 0,5 % en poids, de préférence 0,0005 à 0,1 % en poids et de façon particulièrement préférée 0,001 à 0,02 % en poids d'au moins un composé de métal de transition,
dans chaque cas par rapport à la masse totale du matériau.

14. Composition polymérisable par voie radicalaire selon l'une quelconque des revendications 6 à 13, destinée à l'utilisation thérapeutique dans la restauration de dents endommagées, de préférence en tant que ciment dentaire, matériau temporaire, composite d'obturation ou matériau de scellement.

15. Utilisation non thérapeutique de la composition polymérisable par voie radicalaire selon l'une quelconque des revendications 6 à 13, pour la production ou la réparation de restaurations dentaires, telles que prothèses, dents artificielles, inlays, onlays, couronnes, bridges et prothèses dentaires complètes.
